# EUROPEAN PATENT APPLICATION

(11) **EP 3 031 470 A2**
(43) Date of publication of application: **15.06.2016**
(21) Application number: 16152994.6
(22) Date of filing: 11.09.2006
(51) Int. Cl.: A61K 39/395, C07K 14/475, C07K 16/22, A61K 38/00, A61K 48/00, C12N 15/85, G01N 33/50, A01K 67/027

(54) **MANIPULATION OF REGULATORY T CELL AND DC FUNCTION BY TARGETING NEURITIN GENE USING ANTIBODIES, AGONISTS AND ANTAGONISTS**

(30) Priority: 09.09.2005 US 715766 P
(62) Divisional of application: 06814458.3
(71) Applicant: The Johns Hopkins University, Baltimore, MD 21218 (US)
(72) Inventor: YU, Hong, Baltimore, MD 21210 (US); PARDOLL, Drew, Brookeville, MD 20833 (US); PAN, Xiaoya, Cockeysville, MD 21030 (US); DRAKE, Charles, George, Baltimore, MD 21212 (US); POWELL, Jonathan, D, Baltimore, MD 21212 (US); HUANG, Ching-Tai, 10617 / TW Taipei (TW); BARBI, Joseph, Baltimore, MD 21230 (US); PAN, Fan, Baltimore, MD 21212 (US)
(74) Representative: Shaw, Daniel John

(57) **Abstract**

We demonstrate herein that neuritin controls the homeostasis of regulatory T cells in an antigen dependent manner. Based on this discovery, we describe herein the application of neuritin as a therapeutic agent to manipulate antigen specific regulatory T cells in various disease settings is described. Thus manipulation of Treg cells and DCs through neuritin can be used to enhance immunotherapy of autoimmune diseases, cancer and infectious diseases, as well as enhance lymphocyte engraftment in settings of donor lymphocyte infusion, bone marrow transplant, as well as other types of transplants, and adoptive transfer.

SEQUENCE LISTING

<110> THE JOHNS HOPKINS UNIVERSITY

<120> MANIPULATION OF REGULATORY T CELL AND DC FUNCTION BY
      TARGETING NEURITIN GENE USING ANTIBODIES, AGONISTS AND
      ANTAGONISTS

<130> P/60575.EP02

<150> EP 06814458.3
<151> 2006-09-11

<150> PCT/US2006/035324
<151> 2006-09-11

<150> US 60/715,766
<151> 2005-09-09

<160> 3

<170> PatentIn Ver. 3.3

<210> 1
<211> 429
<212> DNA
<213> Mus musculus

<400> 1
atgggactta agttgaacgg cagatatatt tcactgatcc tcgcggtgca aatagcttac 60
ctggtgcagg ccgtgagagc agcgggcaag tgtgatgcag tctttaaggg cttttcagac 120
tgtttgctca agctgggcga cagcatggcc aactacccgc agggcctgga cgacaagacg 180
aacatcaaga ccgtgtgcac atactgggag gatttccaca gctgcacggt cacagctctt 240
acggattgcc aggaaggggc gaaagatatg tgggataaac tgagaaaaga atcgaaaaac 300
ctcaacatcc aaggcagctt attcgaactc tgcggcagca gcaacggggc ggcggggtcc 360
ctgctcccgg cgctttccgt gctcctggtg tctctctcgg cagctttagc gacctggttt 420
tccttctga                                                         429

<210> 2
<211> 142
<212> PRT
<213> Homo sapiens

<400> 2
Met Gly Leu Lys Leu Asn Gly Arg Tyr Ile Ser Leu Ile Leu Ala Val
  1               5                  10                  15

Gln Ile Ala Tyr Leu Val Gln Ala Val Arg Ala Ala Gly Lys Cys Asp
             20                  25                  30

Ala Val Phe Lys Gly Phe Ser Asp Cys Leu Leu Lys Leu Gly Asp Ser
         35                  40                  45

Met Ala Asn Tyr Pro Gln Gly Leu Asp Asp Lys Thr Asn Ile Lys Thr
     50                  55                  60

Val Cys Thr Tyr Trp Glu Asp Phe His Ser Cys Thr Val Thr Ala Leu
 65                  70                  75                  80

Thr Asp Cys Gln Glu Gly Ala Lys Asp Met Trp Asp Lys Leu Arg Lys
                 85                  90                  95

Glu Ser Lys Asn Leu Asn Ile Gln Gly Ser Leu Phe Glu Leu Cys Gly
            100                 105                 110

Ser Gly Asn Gly Ala Ala Gly Ser Leu Leu Pro Ala Phe Pro Val Leu
        115                 120                 125

Leu Val Ser Leu Ser Ala Ala Leu Ala Thr Trp Leu Ser Phe
    130                 135                 140


<210> 3
<211> 142
<212> PRT
<213> Mus musculus

<400> 3
Met Gly Leu Lys Leu Asn Gly Arg Tyr Ile Ser Leu Ile Leu Ala Val
  1               5                  10                  15

Gln Ile Ala Tyr Leu Val Gln Ala Val Arg Ala Ala Gly Lys Cys Asp
             20                  25                  30

Ala Val Phe Lys Gly Phe Ser Asp Cys Leu Leu Lys Leu Gly Asp Ser
         35                  40                  45

Met Ala Asn Tyr Pro Gln Gly Leu Asp Asp Lys Thr Asn Ile Lys Thr
     50                  55                  60

Val Cys Thr Tyr Trp Glu Asp Phe His Ser Cys Thr Val Thr Ala Leu
 65                  70                  75                  80

Thr Asp Cys Gln Glu Gly Ala Lys Asp Met Trp Asp Lys Leu Arg Lys
                 85                  90                  95

Glu Ser Lys Asn Leu Asn Ile Gln Gly Ser Leu Phe Glu Leu Cys Gly
            100                 105                 110

Ser Ser Asn Gly Ala Ala Gly Ser Leu Leu Pro Ala Leu Ser Val Leu
        115                 120                 125

Leu Val Ser Leu Ser Ala Ala Leu Ala Thr Trp Phe Ser Phe
    130                 135                 140

## Description

### FIELD OF THE INVENTION

The present invention relates to methods and products for the modulation of neuritin mediated immune responses. This modulation can result in the inhibition or stimulation of certain immune response processes.

### BACKGROUND OF THE INVENTION

Regulatory T cells (Treg) have now been described in a large number of systems and have emerged as a major mechanism for the maintenance of self-tolerance and protection from autoimmune disease. Treg cells, characterized as CD4+/CD25+ in most systems, can either emerge from the thymus (so called "natural Treg") or can differentiate from mature T cells under circumstances of peripheral tolerance induction (so called "induced Treg"). Regulatory T cells (Treg) limit autoimmunity but also attenuate the potency of anti-tumor immunity. Enhancement of Treg functions can be used to treat autoimmune diseases while inhibition or elimination of Treg cells can enhance immunotherapy of cancer and infectious diseases.

Accordingly, methods and compositions for the modulation of Treg activity would be useful in the treatment of diseases and disorders characterized by Treg activity, e.g., cancer, infectious disease and immune response.

### SUMMARY

The inventors have demonstrated that neuritin controls the homeostasis of regulatory T cells in an antigen dependent manner. Based on this discovery, neuritin, fragments of neuritin, neuritin agonists, neuritin antagonists, antibodies to neuritin, and neuritin nucleic acid molecules or complements thereof, can be used as a therapeutic agent to manipulate antigen specific regulatory T cells in various disease settings. Thus manipulation of Treg cells and DCs through neuritin can be used, for example, to enhance immunotherapy of autoimmune diseases, cancer and infectious diseases, as well as enhance lymphocyte engraftment in settings of donor lymphocyte infusion, bone marrow transplant, as well as other types of transplants, and adoptive transfer. Moreover, neuritin can be used to selectively enhance or deplete a cell population of T cells that express neuritin.

In one embodiment, the application of Neuritin based immunotherapy modulating Treg cell function is described. The inventors have shown the unique expression of Neuritin in the natural T cell regulatory cells (Treg) and anergic T cells, but not in naive or activated T cells. These findings identify Neuritin as a Treg specific surface molecule for anergic T cells. Further, it has been demonstrated herein that Neuritin can bind to activated dendritic cells and influence Dendritic cell (DC) function, and that soluble neuritin-Ig fusion protein is capable of mediating its effect on Treg cells. Also, Neuritin conditional knockout mice are provided in which the CD4+ T cells do not express neuritin, and a transgenic mouse in which the CD4+ CD8- T cells and the CD4- CD8+ T cells over-express neuritin, to support the screening and evaluation of Neuritin modulating agents *in vivo* in immunotherapy models, in particular autoimmune, transplantation and cancer immunotherapy models.

One embodiment described herein is an isolated antibody which specifically binds neuritin, wherein said antibody binds human T cell regulatory cells. In one aspect the antibody is a functional fragment of an antibody. In another aspect, the antibody is an antagonistic antibody, while in another aspect the antibody is an agonistic antibody. In another aspect, the antibody is a monoclonal antibody, such as the neuritin specific 1D6 and 1A9 antibodies described herein. In another aspect, the antibody is a chimeric antibody, or further the antibody can be a humanized antibody. Also included are compositions, including pharmaceutical compositions comprising the antibodies described herein.

In another embodiment, soluble neuritin, including human and mouse Neuritin/Ig chimeric fusion proteins, are described herein, as are fragments of human and mouse neuritin, particularly those fragments retaining at least one immunomodulatory activity characteristic of Neuritin. RNA aptamers against mouse and human Neuritin are described herein, including those which specifically inhibit human and/or mouse regulatory T cells and those which specifically activate human and/or mouse regulatory T cells. antisense RNA, siRNA and shRNA molecules are also described herein and are useful in the methods of the invention.

In another embodiment, a viral vector comprising a nucleic acid encoding neuritin or a functional fragment thereof, is described which is capable of transfecting an immune cell, i.e. T cells, including CD4+ T cells and CD4+ regulatory T cells. In another embodiment, a polynucleotide encoding a neuritin specific antibody or fragment thereof, vectors including expression vectors and viral vectors comprising the polynucleotide, and host cells comprising the polynucleotides and vectors are described. In another embodiment, a polynucleotide encoding a soluble neuritin, human and/or mouse neuritin chimeric Immunoglobulin fusion molecules or fragment thereof, vectors including expression vectors and viral vectors comprising the polynucleotide, and host cells comprising the polynucleotides and vectors are described.

In another aspect, the invention provides methods of treating autoimmunity. One method of treating an autoimmune disease in an individual by administering Neuritin negative T regulatory cells to the individual, wherein said administering treats said autoimmune disease in said individual. In one embodiment, the method includes isolating Neuritin positive T regulatory cells in vitro by, for example, using antibodies specific for Neuritin. Another method of treating an autoimmune disease in an individual described herein includes administering CD4⁺ T cells containing the neuritin gene, or fragment thereof, wherein the fragment encodes a functionally active fragment, to an individual, in order to treat an autoimmune disease in the individual. Another method of treating an autoimmune disease in an individual described herein includes administering a Neuritin antagonist to an individual, in order to treat the autoimmune disease in the individual. Any of these methods of treating autoimmune disease can be combined with the administration of other agents effective in treating the autoimmune disease.

In another aspect, the invention provides methods of treating. One method of treating cancer in an individual who is receiving as part of the individual's treatment, one or more lymphocyte infusions, includes administering to the individual lymphocyte infusions containing neuritin positive CD4⁺ T cells in order to treat the cancer. Another method of treating cancer described herein applies to an individual who is receiving infusions of tumor specific T cells, and comprises administering depleted neuritin positive CD4⁺ T regulatory cells thereby treat the cancer in the individual. Another method of treating cancer in an individual comprises the administration of soluble neuritin, to reduce the number and/or activity of T reg cells. Any of these methods of treating cancer can be combined with the administration of other agents effective in treating the cancer.

Also described herein are methods of treating an infection in an individual. In one aspect, the infection is treated by administering a soluble neuritin, such as a human Neuritin/Ig chimeric fusion protein, to enhance a vaccine or other therapeutic agent administered to the individual. The infection can be, for example, acute or chronic, including viral infections. Also described herein is a method of increasing tolerance to a transplant in an individual comprising administering an antagonist to neuritin, such as an anti-Neuritin antagonistic antibody, in order to reduce rejection of the graft in the individual. Methods of administering an antagonist of neuritin to treat graft vs. host disease in individuals are described herein, as are methods of enhancing lymphocyte engraftment in an individual who has received a bone marrow transplantation comprising administering an antagonist to neuritin, in order to enhance lymphocyte engraftment. The administration of antagonists of neuritin can also be used to treat inflammatory conditions, such as inflammation of the heart and atherosclerosis. Also described herein is a conditional neuritin knock out mouse, wherein the CD4⁺ T cells of the mouse do not express neuritin, as well as a transgenic mouse, wherein the CD4⁺ CD8- T cells and the CD4⁻ CD8+ T cells of the mouse over-express neuritin.

Also described herein is a method of screening for an agent which modulates the immunomodulatory activity of Neuritin in vitro comprising: in the presence or absence of a test agent, co culturing CD4⁺ T cells which comprise a retroviral vector encoding neuritin and express GFP, e.g., for 24 hours with bone-marrow, e.g., day 7 bone marrow, derived dendritic cells pulsed with an antigen, and comparing the amount of IL-2 production from the supernatant produced after the co culturing in the presence or absence of a test agent, wherein an increase in IL-2 production indicates the test agent inhibits the immunomodulatory activity of Neuritin.

### BRIEF DESCRIPTION OF THE DRAWINGS

The above-recited features, advantages and objects of the invention, as well as others which will become clear, are attained and can be understood in detail, more particular descriptions of the invention briefly summarized above may be had by reference to certain embodiments thereof which are illustrated in the appended drawings. These drawings form a part of the specification. It is to be noted, however, that the appended drawings illustrate preferred embodiments of the invention and therefore are not to be considered limiting in their scope.

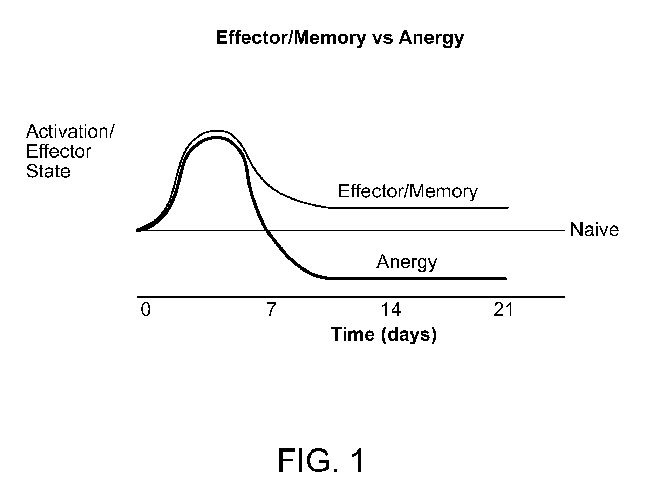

Fig. 1. General view of T cell differentiation into effector/memory vs. anergic phenotypes.

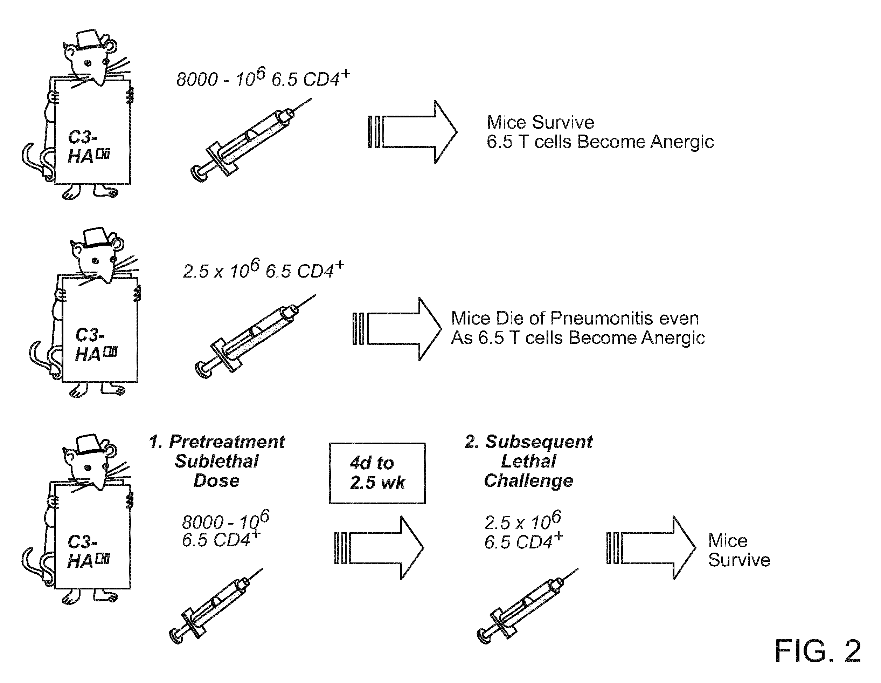

Fig. 2. Induced Treg cells suppress the development of lethal pneumonitis.

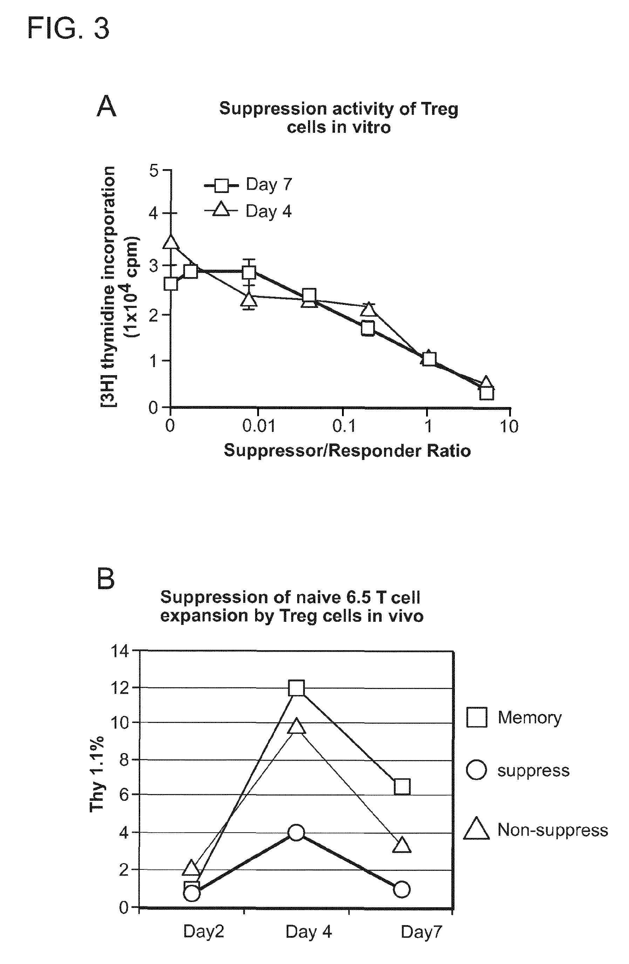

Figs. 3A-B. Anergic 6.5 T cells exhibit regulatory activity in vitro and in vivo. A. In vitro suppression assay. 6.5 T cells adoptive transferred into C3HA host were isolated 4 and 7 days after transfer. Naive 6.5 T cells (responder cells) were stimulated with HA peptide in the presence of different amount of anergic 6.5 cells (suppressor) generated in vivo. B. Anergic 6.5 T cells in C3HA host inhibit the expansion of subsequent transferred 6.5 T cells. T cell expansion was compared after transferring 2 x 10⁶ 6.5 T cells and being activated under three different conditions. Memory condition: 6.5 T cells transferred into B10.D2 host and activate with VacHA. Non-suppression condition: 6.5 T cells were transferred into naive C3HA mice. Suppression condition: 6.5 T cells transferred into C3HA mice that had been transferred with 1 x 10⁵ 6.5 T cells 2 weeks before. The expansion of lethal dose 6.5 T cells were examined 2, 4 and 7 days post the transfer.

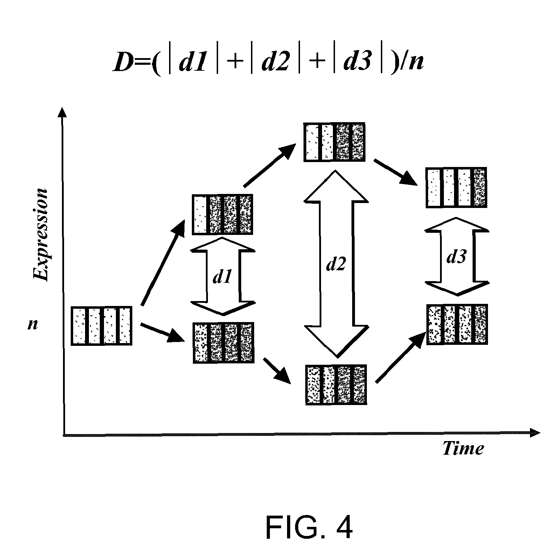

Fig. 4. Ranking the differential expression of genes in CD4⁺ T cells between anergy/Treg induction and effector/memory induction. mRNA prepared from purified naive 6.5 clonotypic CD4⁺ T cells, anergic/Treg and effector/memory 6.5 clonotypic CD4⁺ T cells on various days after adoptive transfer was analyzed by Affymetrix gene chips. The differential expression of genes between anergy/Treg induction and effector/memory induction was ranked by distance. The distance (D) was defined as the sum of the absolute differences of expression between anergic T cells and effector/memory T cells on day 2 (|d1|), day 3 (|d2|), and day 4 (|d3|) after adoptive transfer, divided by the value of naive CD4⁺ T cells (n) for normalization.

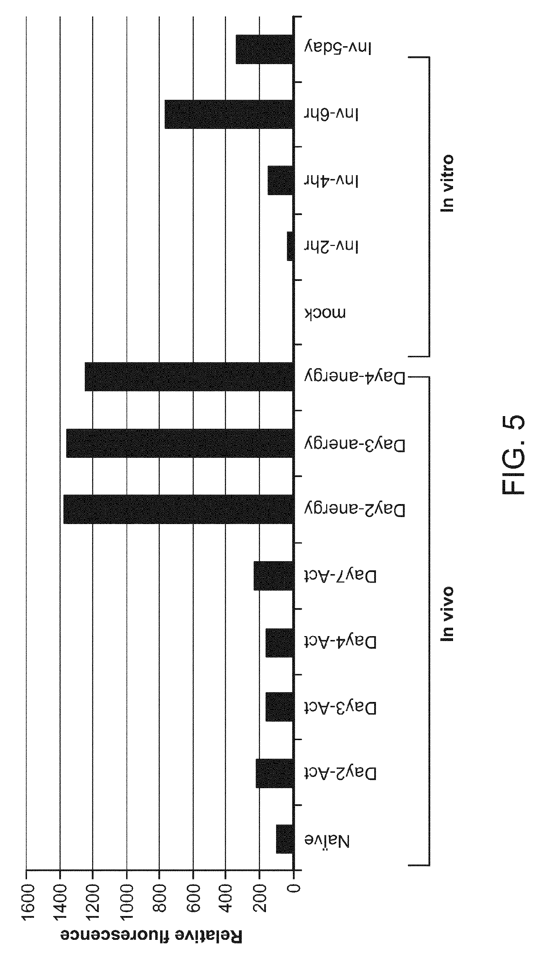

Fig. 5. Genechip data comparing Neuritin expression in anergic vs. activated T cell generated in vivo and in vitro. In vivo, 6.5 TCR transgenic T cells were adoptive transferred into C3HA host and cells were sorted out after 2, 3, 4 days in vivo-anergy condition; or 6.5 cells were transferred into B10D2 host along with VacHA injection-activation condition. In vitro, AE7 cells were anergized by stimulating with anti-CD3 alone for 2, 4, 6hr or stimulate overnight and rest for 5 days in the absence of IL2. RNA samples from these conditions were isolated subjected to cDNA synthesis and Genechip analysis. Data show the relative expression of neuritin from Genechip data.

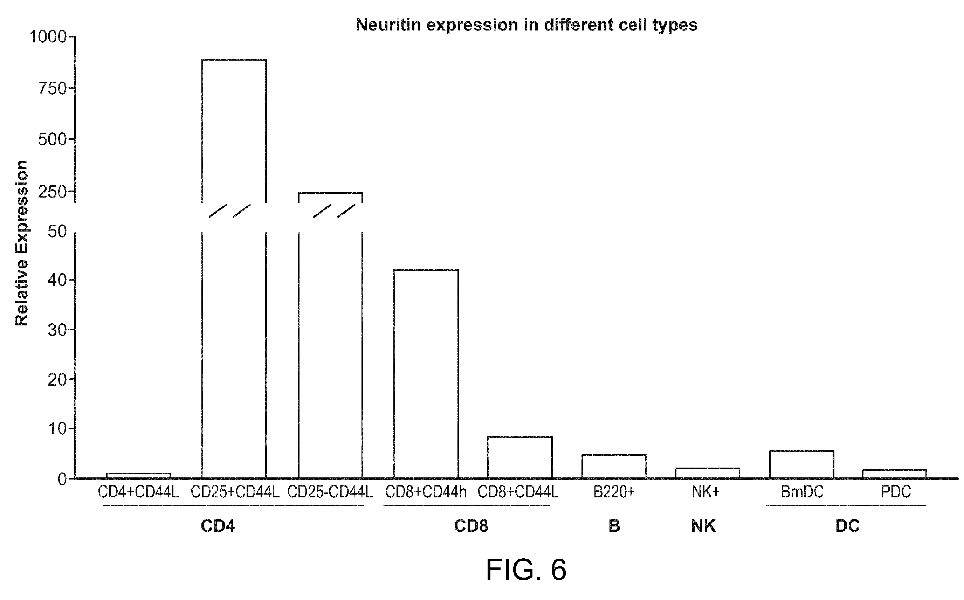

Fig. 6, Comparison of Neuritin expression in various cell types. CD4+CD44lowCD25-, CD4+CD44hiCD25+, CD4+CD44hiCD25-, CD8+CD44hi, CD8+CD44L, B220+CD19+ B cells, DX5+CD3- NK cells, CD11ChiB220- bmDC and CD11C1owB220+ cells are sorted out from lymph node and spleen. RNA was extracted from each cell population and qRT-PCR was carried out on the cDNA. The level of amplification in CD4+CD44L naive T cells is similar to negative control, thus is used as a baseline for comparison.

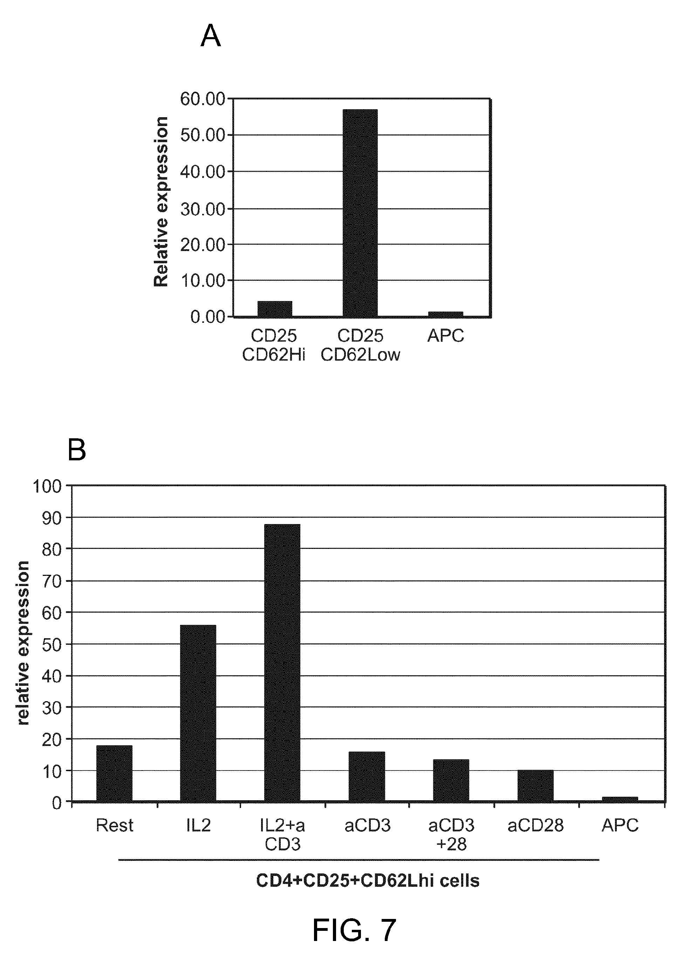

Figs.7A-B. Neuritin is highly expressed in CD4CD25+CD62Low cells and its expression can be induced by IL-2 in CD4CD25+CD62hi cells. qRT-PCR of neuritin expression in sorted sub population of natural Treg cells and upon differential treatment of natural Tregs. **A**. Neuritin Expression is higher in fresh sorted CD4CD25+CD62Low cells than in the CD4CD25+CD62hi cells. **B**. Sorted CD4CD25+CD62hi cells were cultured in the presence of APC alone or with the following treatment: IL-2 (100 u/ml), IL-2 (100 u/ml)+antiCD3(1ug/ml), anti-CD3(1ug/ml)+anti-CD28 (5 ug/ml) or anti-CD28(5ug/ml) alone.

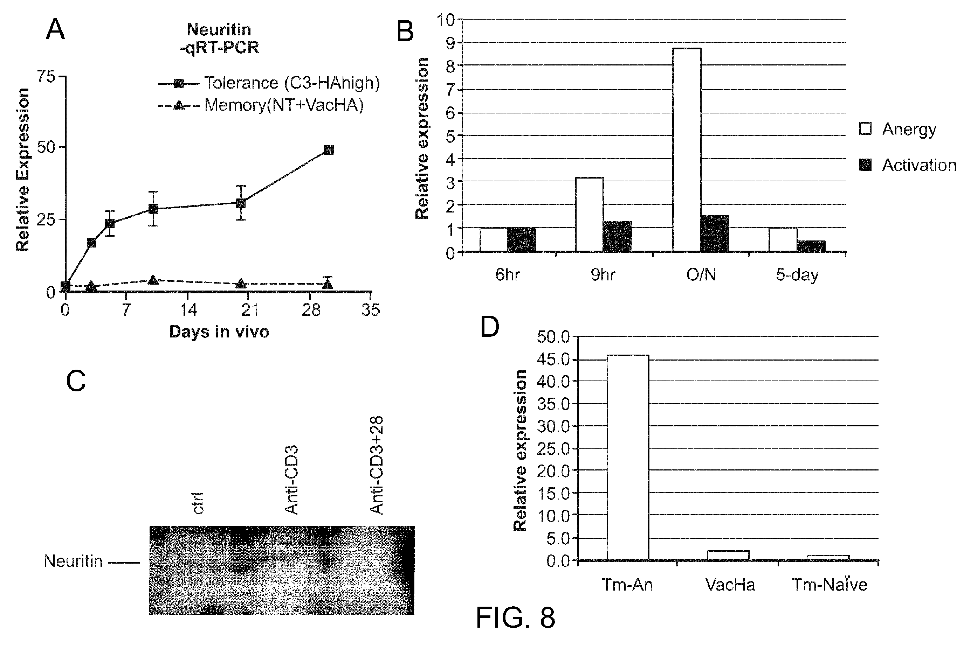

Figs. 8A-D. Expression profile of neuritin under various condition. A. Comparison of neuritin expression in T cells activated under anergic vs. activation condition by qRT-PCR. B. Confirmation of neuritin expression in anergic AE7 cells vs. activated AE7 cells by qRT-PCR. C. Western blotting for neuritin. Cell lysates from anergic AE7 cells, activated or control resting cells. D. qRT-PCR assay on tumor anergized T cells vs. activated cell or control naive cells.

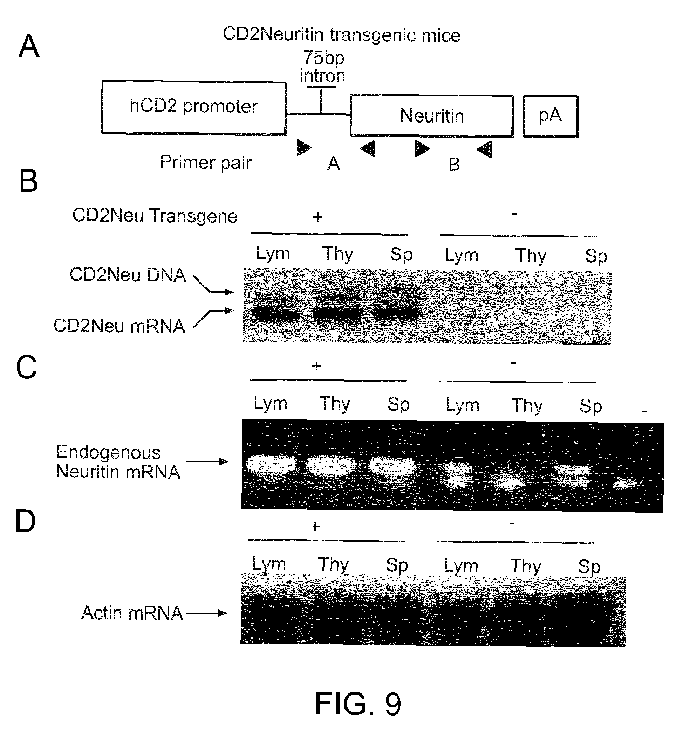

Figs. 9A-C. Generation of CD2Neuritin transgenic mice. A. hCD2 expression construct. B. RNA was extracted from lymph node, thymus and spleen of CD2+neuritin+ and negative littermates. RT-PR was carried out using primer pair A that is specific for the CD2 driven neuritin expression. Amplification of cDNA can be differentiated from the amplification of genomic DNA from the smaller size of the cDNA product due to the spliced out 75bp intron sequence. C. Comparison of Neuritin expression level between CD2Neuritin+ and negative littermates by RT-PCR using Primer pair B that can detect endogenous neuritin expression. D. RT-PCR of actin to control for the amount of cDNA used in the above B. and C. RT-PCR reaction.

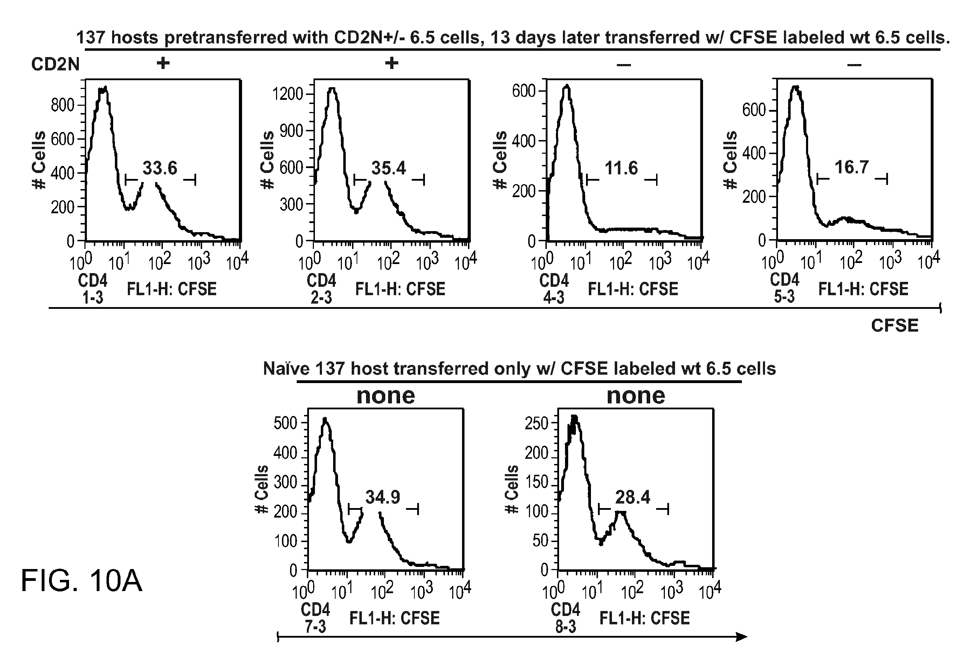

Fig.10A-C. CD2N transgenic T cells show reduced suppression in vivo due to loss of ADT CD2N+ cells. CD2N+ or CD2N- 6.5 TCR+ thy1.1+ total splenocytes were adoptive transferred into C3-HA (137) thy1.2+ HA expressing hosts. 18 days later, CFSE labeled wt 6.5 T cells were ADT again into those and naive C3-HA (137) hosts (without any pretransfer). The expansion of secondary transferred T cells and persistence of cells form the first transfer were examined. A. CFSE dilution of wt 6.5 T cell in host pretransferred w/ CD2N+/- 6.5 cells. B. Persistence of CD2N+ and CD2N- 6.5 cells in C3-HA (137) hosts 21 days post ADT. C. monitor the percentage of transferred CD2N+ and CD2N- cells in C3-HA (137) host overtime.

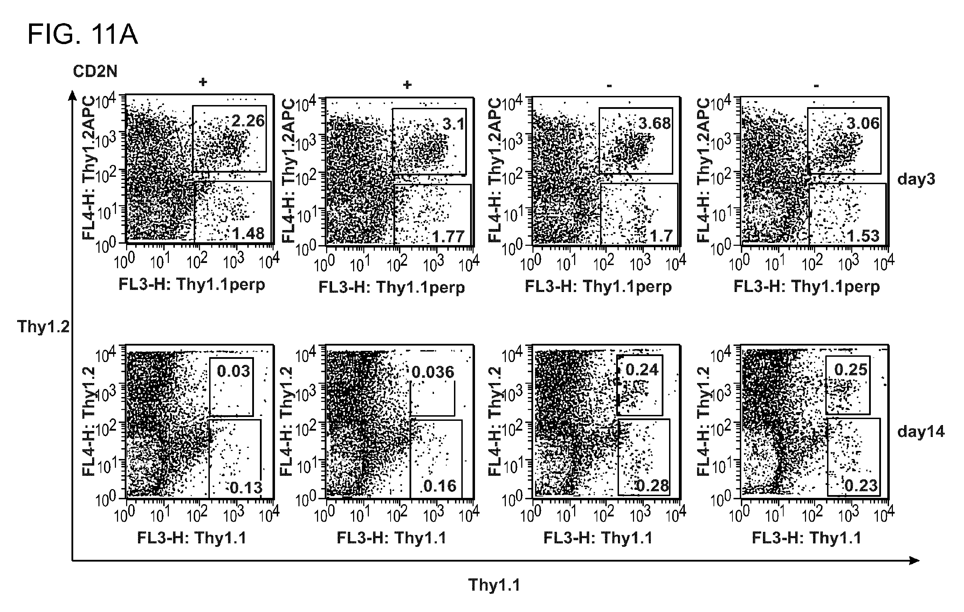

Figs. 11A-B. Soluble neuritin can mediate the loss of Ag-specific T cells in self-Ag expressing hosts. A. CD2N+ or CD2N- 6.5+ thy1.2+thy1.1+ cells were mixed with wt 6.5 thy1.1+ T cells and transferred into C3-HA (137) hosts. Percentage of transferred cells in the spleen were examined 3 and 14 days post ADT. B. WT Thy1.1+6.5+ cells were transferred into C3-HA (137) hosts. ADT hosts were treated w/ NhFc protein or hIgG 200ug ip at the time of ADT and every other day thereafter. The persistence of ADT cells in spleen were examined 13 days post ADT.

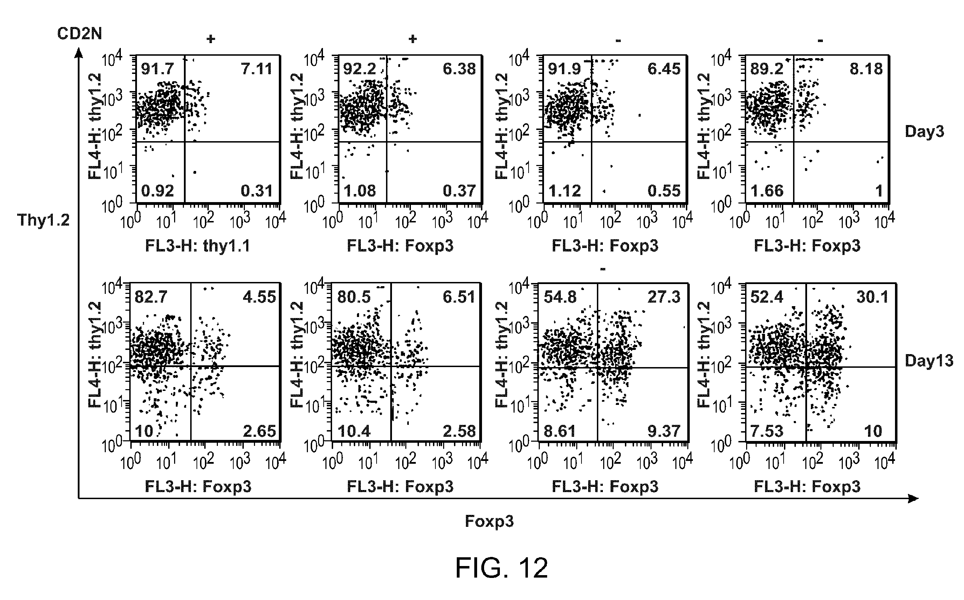

Fig.12. Precipitated Foxp3+ Treg cell loss upon encounter antigen (Foxp3+ regulatory T cells disappear first). CD2N+ or CD2N- 6.5+thy1.1+ splenocytes were transferred into C3-HA (137) hosts. The percentage of foxp3+ cells was examined day3 and 13 post ADT.

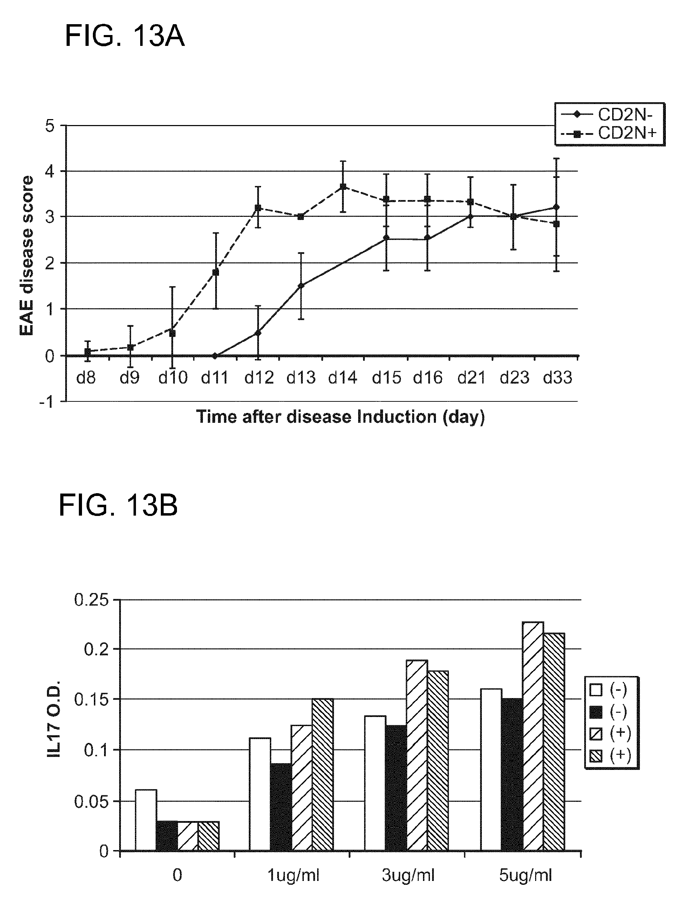

Figs.13A-C. Exacerbated EAE in CD2N+ mice. CD2N+ and littermates on C57/BL6 background were subjected to Mog antigen mediated EAE induction. EAE disease progression were monitored using standard scoring system (A). Draining lymph node and spleen were harvested day10 post disease induction and restimulated in vitro w/ MOG peptide. Supernatant were harvested day2 post stimulation and measured for IL17 secretion (B). Draining lymph node and spleen cells were also restimulated with MOG peptide in the presence of Golgi-Stop for 4hr and stained for IL2 and IFN-g (C).

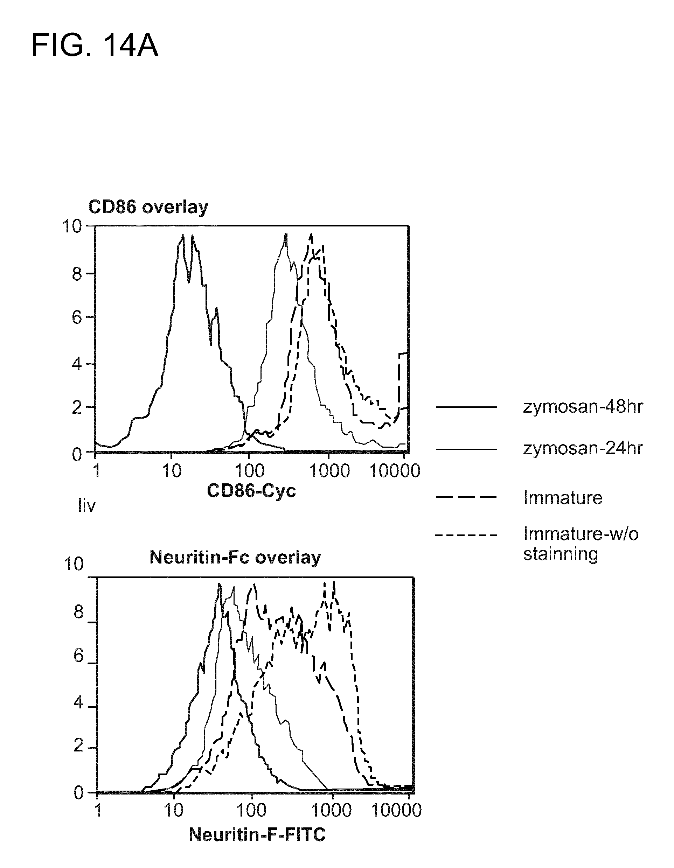

Figs. 14A-B. Neuritin-Fc staining of DC's. A. Bone marrow derived DCs were cultured with or without zymosan for 24 or 48 hours. Cells were subsequently stained for N-Fc. B. Splenic DCs were isolated and cultured in vitro for 24 hours under various conditions, followed by N-Fc staining.

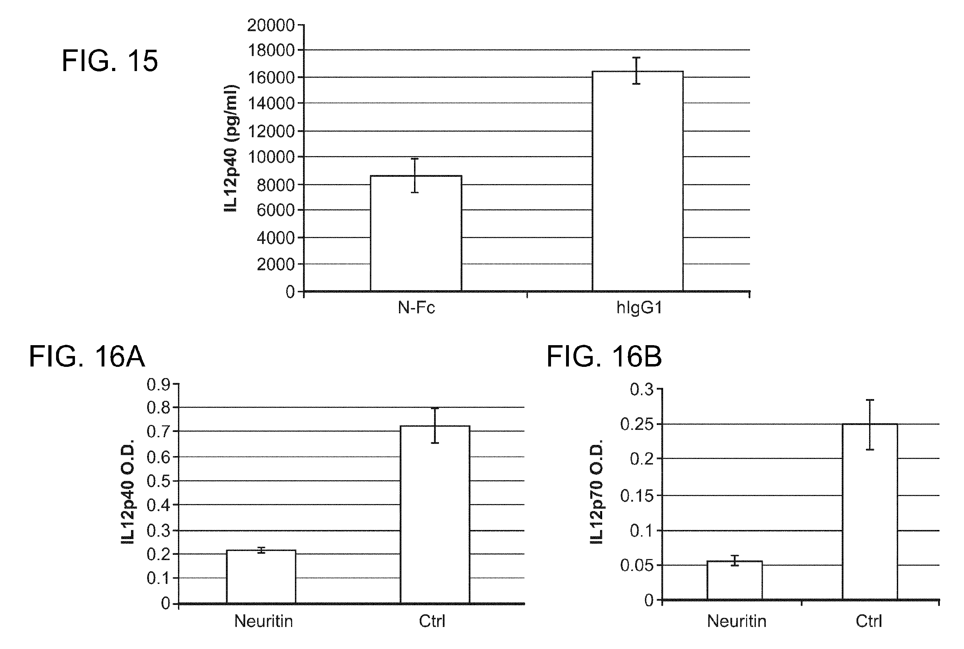

Fig. 15. IL12p40 production from bone marrow derived DCs treated with plate-coated neuritin-Fc (N-Fc) or the control human IgG1 (hIgG1).
Figs. 16A-C. Cytokine production and antigen presentation of DCs treated with T cells with or without neuritin expression. Day 7 Bone marrow derived DCs were pulsed with HA peptide and cocultured with HA specific T cells expressing neuritin and the control vector. 24 hrs after stimulation, IL12p40 and IL12p70 secretion from DCs in the culture supernatant were examined (A and B). DCs were further used to present HA class I specific peptide to HA specific clone 4 CD8 T cells. Proliferation of CD8 T cells were determined 36 hr after stimulation (C).

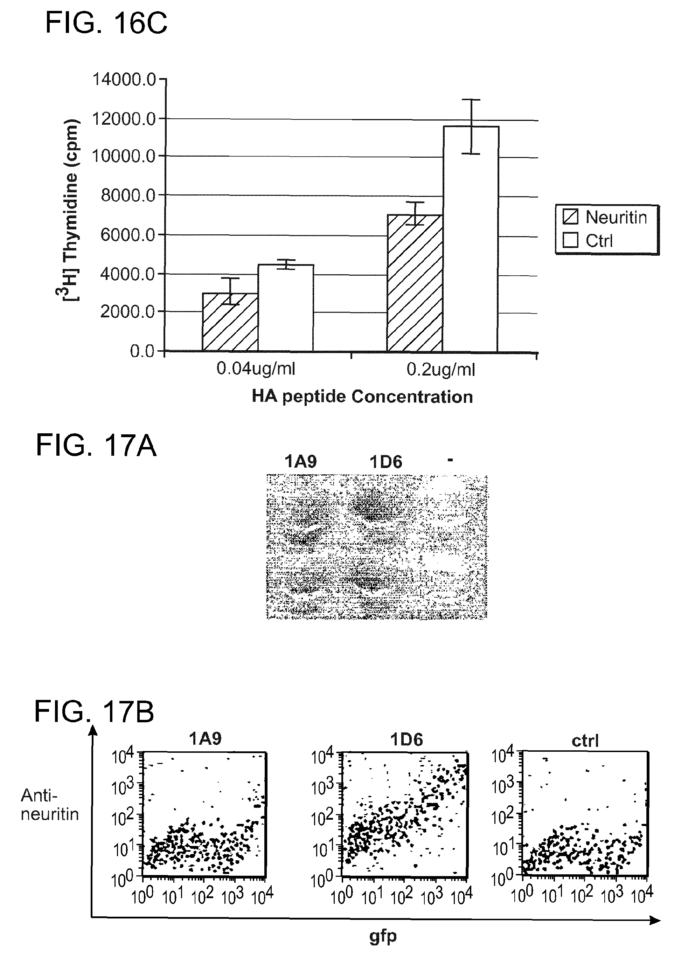

Figs 17A-D. Generation of Neuritin Specific monoclonal antibody. A. two hybridoma clones secrete antibody that can recognize plate-bound neuritin-hFc fusion protein by ELISA. B. Surface staining of 293 cells transfected with full-length neuritin expression construct. Transfected 293 cells are marked by gfp expression. Ctrl vector only expresses GFP. C. 1D6 monoclonal was used in immunoprecipitation from cell lysates and supernatants of 293 cells transfected with full length neuritin (HY10) or soluble neuritin without GPI anchor (HY18). D. Overlay of Neuritin surface staining using the 1D6 monoclonal antibody on CD4+CD8-thymocytes from CD2N+ and negative littermates.

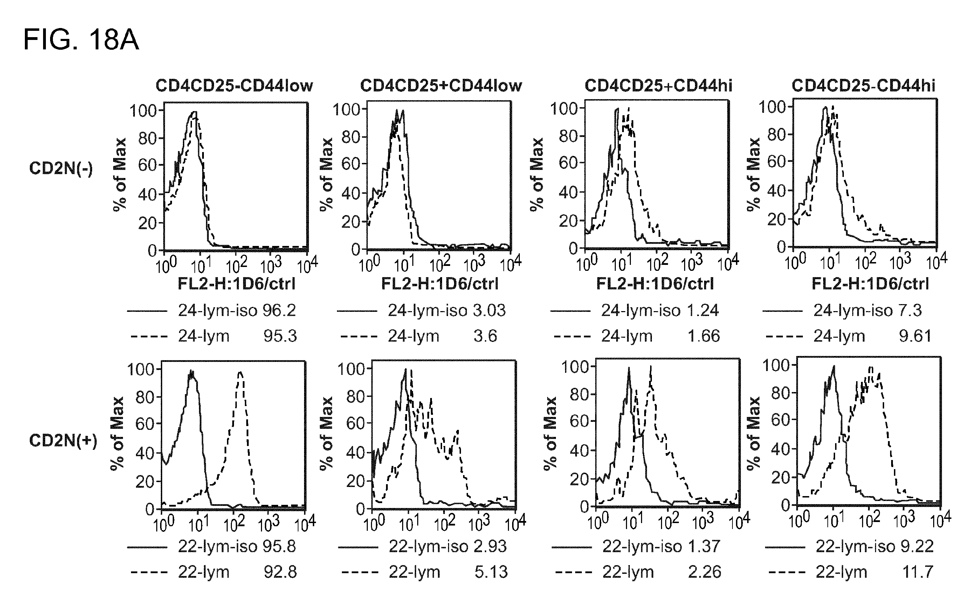

Figs 18A-B. Surface staining of neuritin in subpopulations of CD4 cells from CD2N(+) and control CD2N(-) mice. **A**. Overlay of neuritin and isotype control antibody surface staining in subpopulations of CD4 cells. Cells from peripheral lymph nodes were stained with biotin-labeling 1D6 antibody or the biotin labeled IgG2b isotype control antibody, followed by Avidin-PE, CD4, CD44 and CD25 surface staining. **B**. Overlay of neuritin surface staining on CD4CD25-CD44low, CD4CD25+CD44low, CD4CD25+CD44hi(9), and CD4CD25-CD44hi cells.

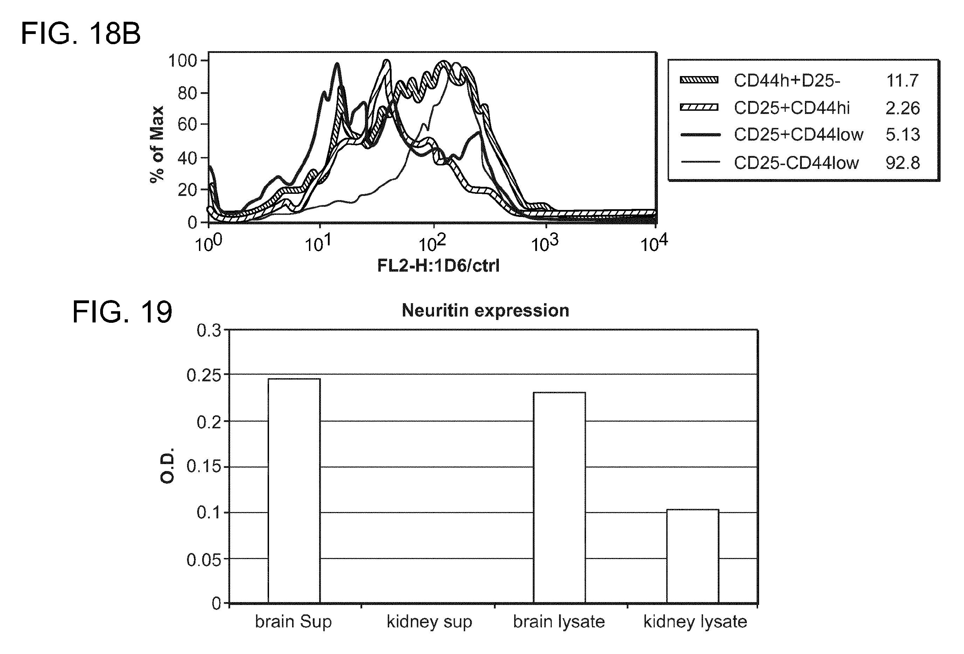

Fig. 19. Sandwich ELISA detecting soluble cell associated neuritin. 25 ug total protein from brain and kidney tissue grinding supernatant and Brain and Kidney cell lysates were applied to ELISA plate coated with 1A9 antibody. The amount of neuritin was detected by biotinylated 1D6 antibody.

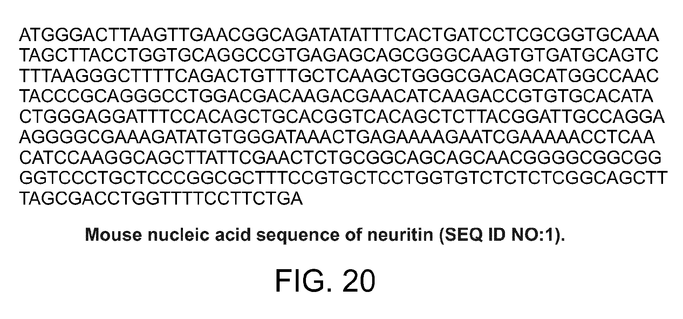

Fig 20 depicts the nucleic acid sequence of mouse neuritin (SEQ ID NO: 1).

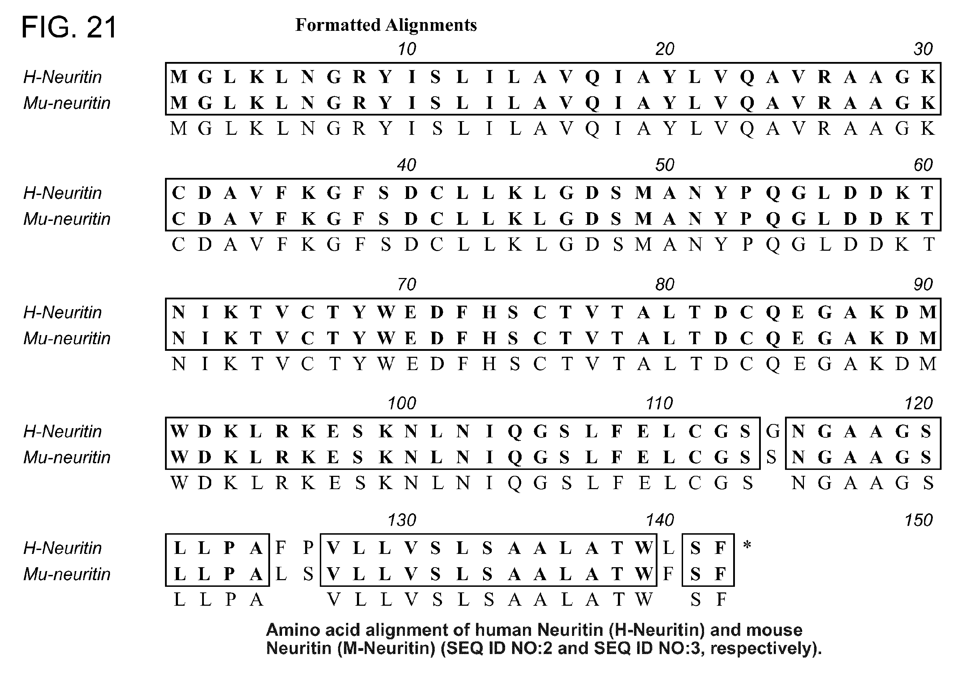

Fig 21 depicts the polypeptide sequences of neuritin from human and mouse (SEQ ID NO:2 and SEQ ID NO:3, respectively).

### DETAILED DESCRIPTION

### Definitions

It is to be noted that the term "a" or "an" entity refers to one or more of that entity; for example, a target-specific compound refers to one or more target-specific compounds. As such, the terms "a" (or "an"), "one or more" and "at least one" can be used interchangeably herein.

As used herein an "aptamer" is a nucleic acid molecule capable of binding to a particular molecule of interest with high affinity and specificity (Tuerk and Gold, Science 249:505 (1990); Ellington and Szostak, Nature 346:818 (1990)). For recent reviews of aptamers and their ligands, see Eaton, Curr. Opin. Chem. Biol. 1:10-16 (1997), Famulok, Curr. Opin. Struct. Biol. 9:324-9(1999), and Hermann and Patel, Science 287:820-5 (2000). Thus, aptamers described herein bind various protein targets, including neuritin, and disrupt the interactions of those proteins with other proteins and/or disrupt signaling by the protein targets. U.S. Pat. No. 5,756,291 discloses DNA aptamers which bind thrombin and inhibit coagulation. For a review of the success of the use of aptamers as therapeutic reagents, see Osborne et al., Curr. Opin. Chem. Biol. 1:5-9 (1997). Aptamers are nucleic acid molecules having specific binding affinity to molecules through interactions other than classic Watson-Crick base pairing. Like antibodies, DNA molecules are able to assume a variety of tridimensional structures depending on their sequence. Described herein are aptamers exhibiting high affinity for neuritin are encompassed.

Also described herein are antibodies that specifically bind to neuritin. These antibodies can be used, e.g., to isolate neuritin, to identify the presence of neuritin, i.e. on anergic T cells, and the like. Antibodies that are antagonistic, i.e. inhibit one or more of the functions of neuritin, such as inhibit or reduce neuritis's T cell regulatory activity, are included, as well as agonist antibodies. To generate antibodies, neuritin polypeptides or peptides (antigenic fragments of neuritin) can be conjugated to another molecule or can be administered with an adjuvant. The coding sequence can be part of an expression cassette or vector capable of expressing the immunogen in vivo (see, e.g., Katsumi (1994) Hum. Gene Ther. 5:1335-9). Methods of producing polyclonal and monoclonal antibodies are known to those of skill in the art and described in the scientific and patent literature, see, e.g., Coligan, Current Protocols in Immunology, Wiley/Greene, NY (1991); Stites (eds.) Basic and Clinical Immunology (7th ed.) Lange Medical Publications, Los Altos, Calif.; Goding, Monoclonal Antibodies:Principle and Practice (2d ed.) Academic Press, New York, N.Y. (1986); Harlow (1988) Antibodies, A Laboratory Manual, Cold Spring Harbor Publications, New York.

Human antibodies can be generated in mice engineered to produce only human antibodies, as described by, e.g., U.S. Pat. Nos. 5,877,397; 5,874, 299; 5,789,650; and 5,939,598. B-cells from these mice can be immortalized using standard techniques (e.g., by fusing with an immortalizing cell line such as a myeloma or by manipulating such B-cells by other techniques to perpetuate a cell line) to produce a monoclonal human antibody - producing cell. See, e.g., U. S. Pat. Nos. 5,916, 771 and 5, 985,615. Antibodies also can be generated in vitro, e.g., using recombinant antibody binding site expressing phage display libraries, in addition to the traditional in vivo methods using animals. See, e.g., Huse Science 246:1275 (1989); Ward Nature 341:544 (1989); Hoogenboom Trends Biotechnol. 15:62-70 (1997); Katz Annu. Rev. Biophys. Biomol. Struct. 26:27-45 (1997).. In addition to intact monoclonal and polyclonal antibodies, various genetically engineered antibodies and antibody fragments (e. g., F(ab') 2, Fab', Fab, Fv, and sFv fragments), and other fragments which retain the antigen binding function and specificity of the parent antibody, can be produced using standard methods. Truncated versions of monoclonal antibodies, for example, can be produced by recombinant methods in which plasmids are generated that express the desired monoclonal antibody fragment(s) in a suitable host. Ladner (U.S. Pat. Nos. 4,946,778 and 4,704,692) describes methods for preparing single polypeptide chain antibodies. Ward et al., Nature 341:544-546, 1989, describes the preparation of heavy chain variable domain which have high antigen-binding affinities. McCafferty et al., Nature 348:552-554, 1990, show that complete antibody V domains can be displayed on the surface of fd bacteriophage, that the phage bind specifically to antigen, and that rare phage (one in a million) can be isolated after affinity chromatography. Boss et al., U. S. Pat. No. 4,816,397, describes various methods for producing immunoglobulins, and immunologically functional fragments thereof, that include at least the variable domains of the heavy and light chains in a single host cell. Cabilly et al., U.S. Pat. No. 4,816,567, describes methods for preparing chimeric antibodies. In addition, the term "antibody" also refers to humanized antibodies in which at least a portion of the framework regions of an immunoglobulin are derived from human immunoglobulin sequences and single chain antibodies as described in U.S. Pat. No. 4,946,778 and to fragments of antibodies such as Fab, F'(ab)2, F v.

As used herein, "antigenic fragments" refers portions of a polypeptide that contains one or more epitopes. Epitopes can be linear, comprising essentially a linear sequence from the antigen, or conformational, comprising sequences which are genetically separated by other sequences but come together structurally at the binding site for the polypeptide ligand. "Antigenic fragments" may be up to any one of 5000, 1000, 500, 400, 300, 200, 100, 50 or 25 or 20 or 10 or 5 amino acids in length.

As used herein, the term "fragment" in the context of a proteinaceous agent refers to a peptide or polypeptide comprising an amino acid sequence of at least 5 contiguous amino acid residues, at least 10 contiguous amino acid residues, at least 15 contiguous amino acid residues, at least 20 contiguous amino acid residues, at least 25 contiguous amino acid residues, at least 40 contiguous amino acid residues, at least 50 contiguous amino acid residues, at least 60 contiguous amino residues, at least 70 contiguous amino acid residues, at least contiguous 80 amino acid residues, at least contiguous 90 amino acid residues, at least contiguous 100 amino acid residues, at least contiguous 125 amino acid residues, at least 150 contiguous amino acid residues, at least contiguous 175 amino acid residues, at least contiguous 200 amino acid residues, or at least contiguous 250 amino acid residues of the amino acid sequence of a polypeptide or a protein. In a specific embodiment, a fragment of a protein or polypeptide retains at least one function of the protein or polypeptide. In another embodiment, a fragment of a protein or polypeptide retains at least one, two, three, four, or five functions of the protein or polypeptide. Preferably, a fragment of an antibody retains the ability to immunospecifically bind to an antigen.

A biologically active portion of a protein, such as neuritin, can be a polypeptide which is, for example, 10, 25, 50, 100 or more amino acids in length. With respect to neuritin, biologically active portions can be used directly as agents that modulate T cell activity or other activities of full length neuritin. Neuritin protein and biologically active fragments thereof, can also be used as targets for developing agents which modulate a neuritin mediated activity, e.g., an activity described herein, and are potential therapeutics for treating disorders associated with insufficient or excessive T cell activity. For example neuritin polypeptides having T cell regulatory activity, including subsequences and sequence variants (e. g., a polypeptide having at least about 65% identity to neuritin), including mimetics etc., having T cell regulatory activity. In addition, the invention provides methods for identifying an agent that modulates an activity of a polypeptide of the invention (e.g., a neuritin polypeptide having T cell regulatory activity), for example the agent may be capable of reducing neuritis's T cell regulatory activities in a cell.

As used herein, the term "fusion protein" refers to a polypeptide that comprises an amino acid sequence of a first protein or polypeptide or fragment thereof, or functional fragment thereof, or an analog or derivative thereof, and an amino acid sequence of a heterologous protein, polypeptide, or peptide (i.e., a second protein or polypeptide or fragment, analog or derivative thereof different than the first protein or fragment, analog or derivative thereof). In one embodiment, a fusion protein comprises a neuritin fused to an IgG molecule.

As used herein, the term "in combination" in reference to therapy refers to the use of more than one therapies (e.g., more than one prophylactic agent and/or therapeutic agent). The use of the term "in combination" does not restrict the order in which therapies (e.g., prophylactic and/or therapeutic agents ) are administered to a subject. A first therapy (e.g., a first prophylactic or therapeutic agent) can be administered prior to (e.g., 5 minutes, 15 minutes, 30 minutes, 45 minutes, 1 hour, 2 hours, 4 hours, 6 hours, 12 hours, 24 hours, 48 hours, 72 hours, 96 hours, 1 week, 2 weeks, 3 weeks, 4 weeks, 5 weeks, 6 weeks, 8 weeks, or 12 weeks before), concomitantly with, or subsequent to (e.g., 5 minutes, 15 minutes, 30 minutes, 45 minutes, 1 hour, 2 hours, 4 hours, 6 hours, 12 hours, 24 hours, 48 hours, 72 hours, 96 hours, 1 week, 2 weeks, 3 weeks, 4 weeks, 5 weeks, 6 weeks, 8 weeks, or 12 weeks after) the administration of a second therapy (e.g., a second prophylactic or therapeutic agent) to a subject.

As used herein, the term "selectively binds" or specifically binds" in the context of proteins encompassed by the invention refers to the specific interaction of any two of a peptide, a protein, a polypeptide, and an antibody, wherein the interaction preferentially occurs as between any two of a peptide, protein, polypeptide and antibody preferentially as compared with any other peptide, protein, polypeptide and antibody. For example, when the two molecules are protein molecules, a structure on the first molecule recognizes and binds to a structure on the second molecule, rather than to other proteins. "Selective binding", "Selective binding", as the term is used herein, means that a molecule binds its specific binding partner with at least 2-fold greater affinity, and preferably at least 10-fold, 20-fold, 50-fold, 100-fold or higher affinity than it binds a non-specific molecule.

As used herein, the terms "subject" and "patient" and "individual" are used interchangeably to refer to an animal (e.g., a mammal, a fish, an amphibian, a reptile, a bird and an insect). In a specific embodiment, a subject is a mammal (e.g., a non-human mammal and a human). In another embodiment, a subject is a pet (e.g., a dog, a cat, a guinea pig, a monkey and a bird), a farm animal (e.g., a horse, a cow, a pig, a goat and a chicken) or a laboratory animal (e.g., a mouse and a rat). In another embodiment, a subject is a primate (e.g., a chimpanzee and a human). In another embodiment, a subject is a human.

As used herein, the terms "therapeutic agent" and "therapeutic agents" refer to any compound(s) which can be used in the treatment, management or amelioration of a disease or condition, or one or more symptoms thereof.

As used herein, the term "therapeutically effective amount" refers to that amount of a therapy (e.g., a therapeutic agent) sufficient to result in the amelioration a disease or condition, or one or more symptoms thereof, prevent advancement of a disease or condition, cause regression of the disease or condition, or to enhance or improve the therapeutic effect(s) of another therapy (e.g., therapeutic agent).

As used herein, the terms "treat", "treatment" and "treating" refer to the reduction or amelioration of the progression, severity and/or reoccurrence of the disease or condition, or one or more symptoms thereof resulting from the administration of one or more compounds identified in accordance the methods of the invention, or a combination of one or more compounds identified in accordance with the invention and another therapy.

As used herein, the terms "cancer", "hyperproliferative" and "neoplastic" refer to cells having the capacity for autonomous growth, i.e., an abnormal state or condition characterized by rapidly proliferating cell growth. Hyperproliferative and neoplastic disease states may be categorized as pathologic, i.e., characterizing or constituting a disease state, or may be categorized as non-pathologic, i.e., a deviation from normal but not associated with a disease state. The term is meant to include all types of cancerous growths or oncogenic processes, metastatic tissues or malignantly transformed cells, tissues, or organs, irrespective of histopathologic type or stage of invasiveness. The terms "cancer" or "neoplasms" include malignancies of the various organ systems, such as affecting lung, breast, thyroid, lymphoid, gastrointestinal, and genito-urinary tract, as well as adenocarcinomas which include malignancies such as most colon cancers, renal-cell carcinoma, prostate cancer and/or testicular tumors, non-small cell carcinoma of the lung, cancer of the small intestine and cancer of the esophagus.

Hematopoietic neoplastic disorders includes diseases involving hyperplastic/neoplastic cells of hematopoietic origin, e.g., arising from myeloid, lymphoid or erythroid lineages, or precursor cells thereof. Typically, the diseases arise from poorly differentiated acute leukemias, e.g., erythroblastic leukemia and acute megakaryoblastic leukemia. Additional exemplary myeloid disorders include, but are not limited to, acute promyeloid leukemia (APML), acute myelogenous leukemia (AML) and chronic myelogenous leukemia (CML) (reviewed in Vaickus, L. (1991) Crit Rev. in Oncol./Hemotol. 11:267-97); lymphoid malignancies include, but are not limited to acute lymphoblastic leukemia (ALL) which includes B- lineage ALL and T-lineage ALL, chronic lymphocytic leukemia (CLL), prolymphocytic leukemia (PLL), hairy cell leukemia (HLL) and Waldenstrom's macroglobulinemia (WM). Additional forms of malignant lymphomas include, but are not limited to non-Hodgkin lymphoma and variants thereof, peripheral T cell lymphomas, adult T cell leukemia/lymphoma (ATL), cutaneous T-cell lymphoma (CTCL), large granular lymphocytic leukemia (LGF), Hodgkin's disease and Reed- Sternberg disease.

The term "autoimmune condition" or "autoimmune disease" means a disease state caused by an inappropriate immune response that is directed to a self-encoded entity which is known as an autoantigen. Autoimmune diseases include, but are not limited to: multiple sclerosis, type-I diabetes, Hashimoto's thyroiditis, Crohn's disease, rheumatoid arthritis, gastritis, autoimmune hepatitis, hemolytic anemia, autoimmune hemophilia, autoimmune lymphoproliferative syndrome (ALPS), autoimmune uveoretinitis, glomerulonephritis, Guillain-Barre syndrome, psoriasis, myasthenia gravis, autoimmune encephalomyelitis, Goodpasture's syndrome, Grave's disease, paraneoplastic pemphigus, autoimmune thrombocytopenic purpura, scleroderma with anti-collagen antibodies, mixed connective tissue disease, pernicious anemia, polymyositis, idiopathic Addison's disease, autoimmune-associated infertility, bullous pemphigoid, Sjogren's syndrome, idiopathic myxedema, colitis and neuroprotection. In certain embodiments, the dosing regimen of such method of invention comprises subcutaneous administration. In another embodiment, a disease treatable by the method of present invention is selected from: allergy, asthma, eczema, hay fever, HVGD or GVHD, and systemic lupus erythematosus (SLE). In certain embodiments, the dosing regimen of such method comprises subcutaneous administration or transcutaneous administration. In one particular embodiment, such autoimmune disease is multiple sclerosis. In yet more particular embodiment, Multiple sclerosis is relapsing-remitting multiple sclerosis. In another embodiment, the autoimmune condition is the rejection of an organ after an organ transplantation.

The terms "disorders" and "diseases" and "condition" are used inclusively and refer to any deviation from the normal structure or function of any part, organ or system of the body (or any combination thereof). A specific disease is manifested by characteristic symptoms and signs, including biological, chemical and physical changes, and is often associated with a variety of other factors including, but not limited to, demographic, environmental, employment, genetic and medically historical factors. Certain characteristic signs, symptoms, and related factors can be quantitated through a variety of methods to yield important diagnostic information.

siRNA is a homologous double stranded RNA that specifically target a gene's product, resulting in null or hypomorphic phenotypes. The use of RNA interference (RNAi) to inhibit neuritin expression, e.g., in CD4+ T cells for example, is described herein. RNAi is a cellular process resulting in enzymatic cleavage and breakdown of mRNA, guided by sequence- specific double-stranded small interfering RNAs (siRNAs) (Dykxhoorn, D. M. et al.; Nat Rev Mol Cell Biol 2003; 4:457-467). Cell transfection with siRNAs results in the generation of a cytoplasmatically located ribonucleoprotein complex called RNA-induced silencing complex. Upon activation of this complex by discarding one of the siRNA strands (Khvorova, A., et al.; Cell 2003; 115:209-216; Schwarz, D. S., et al.; Cell 2003; 115:199-208), the remaining strand targets RISC to complementary RNA sequences leading to the endonucleolytic cleavage of the target RNA by the RISC component Ago-2 (Meister, G., et al.; Mol Cell 2004; 15:185-197; Rand, T. A., et al.; Proc Natl Acad Sci USA 2004; 101: 14385-14389; Song, J. J., et al.; Science 2004; 305:1434-1437). Exogenously added synthetic siRNAs were shown to act as very potent and sequence-specific agents to silence gene expression (Elbashir, S. M., et al.; Nature 2001; 411:494-498), demonstrating the great potential not only for the analysis of gene function but also for gene-specific therapeutic approaches (Cheng, J. C., Moore, T. B., and Sakamoto, K. M.; Mol Genet Metab 2003; 80:121-128; Heidenreich, O. Cum Pharm Biotechnol 2004; 5:349-354). Described herein is RNAi to specifically inhibit neuritin gene expression in immune cells, such as CD4 positive T cells. Gene expression can also be controlled through the use of short hairpin RNA (shRNA) molecules which folds back on themselves to produce the requisite double-stranded portion (see, for example, Yu et al., Proc. Natl. Acad. Sci. USA 99:6047 (2002)).

Polypeptides and peptides of the invention can be isolated from natural sources, be synthetic, or be recombinantly generated polypeptides. Peptides and proteins can be recombinantly expressed in vitro or in vivo. The peptides and polypeptides of the invention can be made and isolated using any method known in the art. Polypeptide and peptides of the invention can also be synthesized, whole or in part, using chemical methods well known in the art. See e.g., Caruthers (1980) Nucleic Acids Res. Symp. Ser. 215-223; Horn (1980) Nucleic Acids Res. Symp. Ser. 225- 232; Banga, A. K., Therapeutic Peptides and Proteins, Formulation, Processing and Delivery Systems (1995) Technomic Publishing Co., Lancaster, Pa. For example, peptide synthesis can be performed using various solid-phase techniques (see e.g., Roberge (1995) Science 269:202; Merrifield (1997) Methods Enzymol. 289:3-13) and automated synthesis may be achieved, e.g., using the ABI 431A Peptide Synthesizer (Perkin Elmer). The skilled artisan will recognize that individual synthetic residues and polypeptides incorporating mimetics can be synthesized using a variety of procedures and methodologies, which are well described in the scientific and patent literature, e.g., Organic Syntheses Collective Volumes, Gilman, et al. (Eds) John Wiley & Sons, Inc., NY. Polypeptides incorporating mimetics can also be made using solid phase synthetic procedures, as described, e.g., by Di Marchi, et al. , U.S. Pat. No. 5,422,426. Peptides and peptide mimetics of the invention can also be synthesized using combinatorial methodologies. Various techniques for generation of peptide and peptidomimetic libraries are well known, and include, e.g., multipin, tea bag, and split-couple-mix techniques; see, e.g., al-Obeidi (1998) Mol. Biotechnol. 9:205-223; Hruby (1997) Curr. Opin. Chem. Biol. 1:114-119; Ostergaard (1997) Mol. Divers. 3:17-27; Ostresh (1996) Methods Enzymol. 267:220-234. Modified peptides of the invention can be further produced by chemical modification methods, see, e.g., Belousov (1997) Nucleic Acids Res. 25:3440-3444; Frenkel (1995) Free Radic. Biol. Med. 19:373-380; Blommers (1994) Biochemistry 33:7886-7896. As used herein, "polypeptide sequences encoded by" refers to the amino acid sequences obtained after translation of the protein coding region of a gene, as defined herein.

As used herein, "nucleic acid(s)" is interchangeable with the term "polynucleotide(s)" and it generally refers to any polyribonucleotide or poly-deoxyribonucleotide, which may be unmodified RNA or DNA or modified RNA or DNA or any combination thereof. "Nucleic acids" include, without limitation, single- and double-stranded nucleic acids. As used herein, the term "nucleic acid(s)" also includes DNAs or RNAs as described above that contain one or more modified bases. Thus, DNAs or RNAs with backbones modified for stability or for other reasons are "nucleic acids". The term "nucleic acids" as it is used herein embraces such chemically, enzymatically or metabolically modified forms of nucleic acids, as well as the chemical forms of DNA and RNA characteristic of viruses and cells, including for example, simple and complex cells. A "nucleic acid" or "nucleic acid sequence" may also include regions of single- or double-stranded RNA or DNA or any combinations thereof.

The terms percent "sequence identity" or "sequence homology," in the context of two or more nucleic acids or polypeptide sequences, refers to sequences or subsequences thereof that have a specified percentage of nucleotides (or amino acid residues) that are the same, when compared and aligned for maximum correspondence over a comparison window. Percent identity can be measured by manual alignment and visual inspection or using a sequence comparison algorithm, as described herein. This definition also refers to the complement (antisense strand) of a sequence. For example, in various embodiments, polypeptides of the invention include those having an amino acid sequence at least about 70%, at least about 80%, at least about 90%, at least about 95% and at least about 99% identical to an exemplary sequence set forth in SEQ ID NO:2. Polypeptide subsequences are also included. In additional embodiments, a subsequence is at least about 15, 20, 25, 30, 40, 50, 75, 125, 150 or 200, or greater amino acids (e.g., 300, 350, 400, 450, 500, 550, etc.) in length. Thus, a polypeptide sequence having the requisite sequence identity to SEQ ID NO:2, or a subsequence thereof, also is a polypeptide of the invention. In various additional embodiments, the polypeptides, including subsequences have one or more activities of a sequence such as that of neuritin.

As used herein, a "nucleic acid probe" includes nucleic acids capable of binding to a complementary sequence of a nucleic acid member on an array through sets of non-covalent bonding interactions, including complementary base pairing interactions. As used herein, a nucleic acid probe may include natural (i. e., A, G, C, or T) or modified bases (7-deazaguanosine, inosine, etc.). In addition, the bases in nucleic acid probes may be joined by a linkage other than a phosphodiester bond, so long as it does not interfere with hybridization (i.e., the nucleic acid probe still specifically binds to its complementary sequence under standard stringent or selective hybridization conditions). Thus, nucleic acid probes may be peptide nucleic acids in which the constituent bases are joined by peptide bonds rather than phosphodiester linkages.

The term, "primer", as used herein refers to an oligonucleotide, whether occurring naturally as in a purified restriction digest or produced synthetically, which is capable of acting as a point of initiation of synthesis when placed under conditions in which synthesis of a primer extension product, which is complementary to a nucleic acid strand, is induced, i.e., in the presence of nucleotides and an inducing agent such as a DNA polymerase and at a suitable temperature and pH. The primer may be either single-stranded or double-stranded and must be sufficiently long to prime the synthesis of the desired extension product in the presence of the inducing agent. The exact length of the primer will depend upon many factors, including temperature, source of primer and the method used. For example, for diagnostic applications, depending on the complexity of the target sequence, the oligonucleotide primer typically contains 15-25 or more nucleotides, although it may contain fewer or more nucleotides. The factors involved in determining the appropriate length of primer are readily known to one of ordinary skill in the art. In general, the design and selection of primers embodied by the instant invention is according to methods that are standard and well known in the art, see Dieffenbach, C.W., Lowe, T.M.J., Dveksler, G.S. (1995) General Concepts for PCR Primer Design. In: PCR Primer, A Laboratory Manual (Eds. Dieffenbach, C.W, and Dveksler, G.S.) Cold Spring Harbor Laboratory Press, New York, 133-155; Innis, M.A., and Gelfand, D.H. (1990) Optimization of PCRs. In: PCR protocols, A Guide to Methods and Applications (Eds. Innis, M.A., Gelfand, D.H., Sninsky, J.J., and White, T.J.) Academic Press, San Diego, 3-12; Sharrocks, A.D. (1994) The design of primers for PCR. In: PCR Technology, Current Innovations (Eds. Griffin, H.G., and Griffin, A.M, Ed.) CRC Press, London, 5-11.

Regulatory T cells (Treg) have now been described in a large number of systems and have emerged as a major mechanism for the maintenance of self-tolerance and protection from autoimmune disease. Treg cells, characterized as CD4+/CD25+ in most systems, can either emerge from the thymus (so called "natural Treg") or can differentiate from mature T cells under circumstances of peripheral tolerance induction (so called "induced Treg"). In a number of cases, Treg cells have been shown to limit the efficacy of therapeutic tumor vaccines against established cancers. Treg cells can exert its function through professional antigen presenting cells, such as dendritic cells (DC). Dendritic cells (DCs) are scarce cells in the immune system that are specialized in processing antigens, presenting to naive T lymphocytes, and playing an important role in initiating host immune responses. DCs also play an important role in maintaining tolerance. Therapeutic manipulation of Treg in vivo requires the identification of Treg specific receptors.

Neuritin (SEQ ID NOs:1 and 2) was originally cloned in the neural system in 1997. No known function and expression in the immune system have been described. We have identified the unique regulated expression pattern of neuritin in both natural regulatory T cells and anergic T cells. Using overexpression animal model, we have demonstrated that neuritin controls the homeostasis of regulatory T cells in an antigen dependent manner. Furthermore, soluble neuritin-Ig fusion protein is capable of mediating its effect on Treg cells. These discoveries allow the application of neuritin as a therapeutic agent to manipulate antigen specific regulatory T cells in various disease settings, as described herein.

Autoimmune diseases in an individual can be treated by administering an antagonist of neuritin's immunomodulatory activity. Though not being bound by theory, an antagonist off neuritin prolongs the persistence of antigen specific Treg cells. Exemplary neuritin antagonists include antagonistic antibodies which specifically bind neuritin and inhibit an immunomodulating function of neuritin, neuritin blocking antibodies, RNA aptamers, and antagonist peptides, drugs and molecules.

Treatment of cancer in an individual can be effected by administering soluble neuritin, such as a soluble neuritin fusion protein. Though not being bound by theory, the administered soluble neuritin fusion protein eliminates tumor antigen specific Treg cells and anergized T cells. Co-administration of soluble neuritin with a tumor vaccine can be used to boost the tumor vaccine immune responses, by eliminating tumor antigen specific regulatory T cells.

Chronic infection, such as a chronic viral infection, in an individual can be treated by: administering soluble neuritin to patients receiving vaccine for the infectious agent. Alternatively, chronic infection can be treated by simply administering soluble neuritin-Ig fusion protein to eliminate antigen specific Treg cells, thus boost immune response against the pathogen.

Graft-vs.-host disease can be treated by administering neuritin antagonists to prevent the loss of Treg cells. Administering neuritin antagonists can also be used to reduce graft rejection in patients receiving a transplant. Lymphocyte engraftment after bone marrow transplantation can be enhanced by administering neuritin blocking antibody.

Inflammatory conditions such as atherosclerosis and myocardial infarction can be treated by administering neuritin antagonists.

Immunotherapeutic products include antibodies which specifically bind to Neuritin, and prolong antigen specific T cells in vivo, mouse and human Neuritin-Ig chimeric molecules to eliminate antigen specific regulatory T cells, agonist and antagonist peptides against mouse and human Neuritin that affect Treg cell homeostasis, RNA aptamers against mouse and human Neuritin that affect Treg cell homeostasis, and Retroviral, adeno or other viral vectors to introduce Neuritin into T cells to affect immune responses. Also included are methods to screen for agonist and antagonist drugs affecting mouse and human Neuritin signaling affecting Treg cell homeostasis, as well as the drugs themselves.

The invention thus provides chimeric or fusion proteins. These comprise an neuritin peptide sequence operatively linked to a heterologous peptide having an amino acid sequence not substantially homologous to the neuritin. "Operatively linked" indicates that the neuritin peptide and the heterologous peptide are fused in-frame. The heterologous peptide can be fused to the N-terminus or C-terminus of the neuritin or can be internally located.

In one embodiment the fusion protein does not affect neuritin function per se. For example, the fusion protein can be a GST-fusion protein in which the neuritin sequences are fused to the C-terminus of the GST sequences. Other types of fusion proteins include, but are not limited to, enzymatic fusion proteins, for example beta-galactosidase fusions, yeast two-hybrid GALA fusions, poly-His fusions and Ig fusions. Such fusion proteins, particularly poly-His fusions, can facilitate the purification of recombinant neuritin. In certain host cells (e.g., mammalian host cells), expression and/or secretion of a protein can be increased by using a heterologous signal sequence. Therefore, in another embodiment, the fusion protein contains a heterologous signal sequence at its N-terminus.

EP-A-O 464 533 discloses fusion proteins comprising various portions of immunoglobulin constant regions. The Fc is useful in therapy and diagnosis and thus results, for example, in improved pharmacokinetic properties (EP-A 0232 262). In drug discovery, for example, human proteins have been fused with Fc portions for the purpose of high-throughput screening assays to identify antagonists (Bennett et al. (1995) J. Mol. Recog. 8:52-58 (1995) and Johanson et al. J. Biol. Chem. 270:9459-9471). Thus, this invention also encompasses soluble fusion proteins containing an neuritin polypeptide and various portions of the constant regions of heavy or light chains of immunoglobulins of various subclass (IgG, IgM, IgA, IgE). Preferred as immunoglobulin is the constant part of the heavy chain of human IgG, particularly IgG1, where fusion takes place at the hinge region. For some uses it is desirable to remove the Fc after the fusion protein has been used for its intended purpose, for example when the fusion protein is to be used as antigen for immunizations. In a particular embodiment, the Fc part can be removed in a simple way by a cleavage sequence, which is also incorporated and can be cleaved with factor Xa.

A chimeric or fusion protein can be produced by standard recombinant DNA techniques. For example, DNA fragments coding for the different protein sequences are ligated together in-frame in accordance with conventional techniques. In another embodiment, the fusion gene can be synthesized by conventional techniques including automated DNA synthesizers. Alternatively, PCR amplification of gene fragments can be carried out using anchor primers which give rise to complementary overhangs between two consecutive gene fragments which can subsequently be annealed and re-amplified to generate a chimeric gene sequence (see Ausubel et al. (1992) Current Protocols in Molecular Biology). Moreover, many expression vectors are commercially available that already encode a fusion moiety (e.g., a GST protein). An neuritin-encoding nucleic acid can be cloned into such an expression vector such that the fusion moiety is linked in-frame to the neuritin.

The isolated neuritin protein can be purified from cells that naturally express it, such as from any of those cells identified herein, especially purified from cells that have been altered to express it (recombinant), or synthesized using known protein synthesis methods.

In one embodiment, the protein is produced by recombinant DNA techniques. For example, a nucleic acid molecule encoding the neuritin polypeptide, or fragment thereof, is cloned into an expression vector, the expression vector introduced into a host cell and the protein expressed in the host cell. The protein can then be isolated from the cells by an appropriate purification scheme using standard protein purification techniques. Polypeptides often contain amino acids other than the 20 amino acids commonly referred to as the 20 naturally-occurring amino acids. Further, many amino acids, including the terminal amino acids, may be modified by natural processes, such as processing and other post-translational modifications, or by chemical modification techniques well known in the art. Common modifications that occur naturally in polypeptides are described in basic texts, detailed monographs, and the research literature, and they are well known to those of skill in the art.

Accordingly, the polypeptides also encompass derivatives or analogs in which a substituted amino acid residue is not one encoded by the genetic code, in which a substituent group is included, in which the mature polypeptide is fused with another compound, such as a compound to increase the half-life of the polypeptide (for example, polyethylene glycol), or in which the additional amino acids are fused to the mature polypeptide, such as a leader or secretory sequence or a sequence for purification of the mature polypeptide or a pro-protein sequence.

Known modifications include, but are not limited to, acetylation, acylation, ADP-ribosylation, amidation, covalent attachment of flavin, covalent attachment of a heme moiety, covalent attachment of a nucleotide or nucleotide derivative, covalent attachment of a lipid or lipid derivative, covalent attachment of phosphatidylinositol, cross-linking, cyclization, disulfide bond formation, demethylation, formation of covalent crosslinks, formation of cystine, formation of pyroglutamate, formylation, gamma carboxylation, glycosylation, GPI anchor formation, hydroxylation, iodination, methylation, myristoylation, oxidation, proteolytic processing, phosphorylation, prenylation, racemization, selenoylation, sulfation, transfer-RNA mediated addition of amino acids to proteins such as arginylation, and ubiquitination.

Such modifications are well-known to those of skill in the art and have been described in great detail in the scientific literature. Several particularly common modifications, glycosylation, lipid attachment, sulfation, gamma-carboxylation of glutamic acid residues, hydroxylation and ADP-ribosylation, for instance, are described in most basic texts, such as Proteins-Structure and Molecular Properties, 2nd ed., T.E. Creighton, W. H. Freeman and Company, New York (1993). Many detailed reviews are available on this subject, such as by Wold, F., Posttranslational Covalent Modification of Proteins, B. C. Johnson, Ed., Academic Press, New York 1-12 (1983); Seifter et al. (1990) Meth. Enzymol. 182: 626-646) and Rattan et al. (1992) Ann. N.Y Acad. Sci. 663:48-62).

As is also well known, polypeptides are not always entirely linear. For instance, polypeptides may be circular, with or without branching, generally as a result of post-translation events, including natural processing events and events brought about by human manipulation which do not occur naturally. Circular, branched and branched circular polypeptides may be synthesized by non-translational natural processes and by synthetic methods.

Modifications can occur anywhere in a polypeptide, or polypeptide fragment, including the peptide backbone, the amino acid side-chains and the amino or carboxyl termini. Blockage of the amino or carboxyl group in a polypeptide, or both, by a covalent modification, is common in naturally-occurring and synthetic polypeptides. For instance, the aminoterminal residue of polypeptides made in E. coli, prior to proteolytic processing, almost invariably will be N-formylmethionine.

The modifications can be a function of how the protein is made. For recombinant polypeptides, for example, the modifications will be determined by the host cell posttranslational modification capacity and the modification signals in the polypeptide amino acid sequence. Accordingly, when glycosylation is desired, a polypeptide should be expressed in a glycosylating host, generally a eukaryotic cell. Insect cells often carry out the same posttranslational glycosylations as mammalian cells and, for this reason, insect cell expression systems have been developed to efficiently express mammalian proteins having native patterns of glycosylation. Similar considerations apply to other modifications.

The same type of modification may be present in the same or varying degree at several sites in a given polypeptide. Also, a given polypeptide may contain more than one type of modification.

Neuritin polypeptides are useful for producing antibodies specific for neuritin, regions, or fragments. Regions having a high antigenicity index score can be determined by one of skill in the art using routine methods and software.

The neuritin polypeptides are useful for biological assays related to neuritins. Such assays involve any of the known neuritin functions or activities or properties useful for diagnosis and treatment of neuritin-related conditions such as those identified herein.

The neuritin polypeptides are also useful in drug screening assays, in cell-based or cell-free systems. Cell-based systems can be native, i.e., cells that normally express the neuritin, as a biopsy or expanded in cell culture. In one embodiment, however, cell-based assays involve recombinant host cells expressing the neuritin.

Determrining the ability of the test compound to interact with the neuritin can also comprise determining the ability of the test compound to preferentially bind to the polypeptide as compared to the ability of a known binding molecule to bind to the polypeptide.

The polypeptides can be used to identify compounds that modulate neuritin activity. Such compounds, for example, can increase or decrease affinity or rate of binding to peptide substrate, compete with peptide substrate for binding to the neuritin, or displace peptide substrate bound to the neuritin. Both neuritin and appropriate variants and fragments can be used in high-throughput screens to assay candidate compounds for the ability to bind to the neuritin. These compounds can be further screened against a functional neuritin to determine the effect of the compound on the neuritin activity. Compounds can be identified that activate (agonist) or inactivate (antagonist) the neuritin to a desired degree. Modulatory methods can be performed in vitro (e.g., by culturing the cell with the agent) or, alternatively, in vivo (e.g., by administering the agent to a subject.

The neuritin polypeptides can be used to screen a compound for the ability to stimulate or inhibit interaction between the neuritin protein and a target molecule that normally interacts with the neuritin protein. The assay includes the steps of combining the neuritin protein with a candidate compound under conditions that allow the neuritin protein or fragment to interact with the target molecule, and to detect the formation of a complex between the neuritin protein and the target or to detect the biochemical consequence of the interaction with the neuritin and the target.

Determining the ability of the neuritin to bind to a target molecule can also be accomplished using a technology such as real-time Bimolecular Interaction Analysis (BIA). Sjolander et al. (1991) Anal. Chem. 63:2338-2345 and Szabo et al (1995) Curr. Opin. Struct. Biol. 5:699-705. As used herein, "BIA" is a technology for studying biospecific interactions in real time, without labeling any of the interactants (e.g., BIAcore.TM.). Changes in the optical phenomenon surface plasmon resonance (SPR) can be used as an indication of real-time reactions between biological molecules.

The test compounds of the present invention can be obtained using any of the numerous approaches in combinatorial library methods known in the art, including: biological libraries; spatially addressable parallel solid phase or solution phase libraries; synthetic library methods requiring deconvolution; the 'one-bead one-compound' library method; and synthetic library methods using affinity chromatography selection. The biological library approach is limited to polypeptide libraries, while the other four approaches are applicable to polypeptide, non-peptide oligomer or small molecule libraries of compounds (Lam, K. S. (1997) Anticancer Drug Des. 12:145).

Examples of methods for the synthesis of molecular libraries can be found in the art, for example in DeWitt et al. (1993) Proc. Natl. Acad. Sci. USA 90:6909; Erb et al. (1994) Proc. Natl. Acad. Sci. USA 91:11422; Zuckermann et al. (1994). J. Med. Chem. 37:2678; Cho et al. (1993) Science 261:1303; Carell et al. (1994) Angew. Chem. Int. Ed. Engl. 33:2059; Carell et al. (1994) Angew. Chem. Int. Ed. Engl. 33:2061; and in Gallop et al. (1994) J. Med. Chem. 37:1233. Libraries of compounds may be presented in solution (e.g., Houghten (1992) Biotechniques 13:412-421), or on beads (Lam (1991) Nature 354:82-84), chips (Fodor (1993) Nature 364:555-556), bacteria (Ladner U.S. Pat. No. 5,223,409), spores (Ladner U.S. Pat. No. '409), plasmids (Cull et al. (1992) Proc. Natl. Acad. Sci. USA 89:1865-1869) or on phage (Scott and Smith (1990) Science 249:386-390); (Devlin (1990) Science 249:404-406); (Cwirla et al. (1990) Proc. Natl. Acad. Sci. 97:6378-6382); (Felici (1991) J. Mol. Biol. 222:301-310); (Ladner supra).

Candidate compounds include, for example, 1) peptides such as soluble peptides, including Ig-tailed fusion peptides and members of random peptide libraries (see, e.g., Lam et al. (1991) Nature 354:82-84; Houghten et al. (1991) Nature 354:84-86) and combinatorial chemistry-derived molecular libraries made of D- and/or L- configuration amino acids; 2) phosphopeptides (e.g., members of random and partially degenerate, directed phosphopeptide libraries, see, e.g., Songyang et al. (1993) Cell 72:767-778); 3) antibodies (e.g., polyclonal, monoclonal, humanized, anti-idiotypic, chimeric, and single chain antibodies as well as Fab, F(ab').sub.2, Fab expression library fragments, and epitope-binding fragments of antibodies); and 4) small organic and inorganic molecules (e.g., molecules obtained from combinatorial and natural product libraries).

One candidate compound is a soluble full-length neuritin or fragment that competes for peptide binding. Other candidate compounds include mutant neuritins or appropriate fragments containing mutations that affect neuritin function and compete for peptide substrate. Accordingly, a fragment that competes for substrate, for example with a higher affinity, or a fragment that binds substrate but does not degrade it, is encompassed by the invention.

The invention provides other end points to identify compounds that modulate (stimulate or inhibit) neuritin activity. The assays typically involve an assay of cellular events that indicate neuritin activity. Thus, the expression of genes that are up- or down-regulated in response to the neuritin activity can be assayed. In one embodiment, the regulatory region of such genes can be operably linked to a marker that is easily detectable, such as luciferase. Alternatively, modification of the neuritin could also be measured.

Any of the biological or biochemical functions mediated by neuritin can be used as an endpoint assay. These include all of the biochemical or biochemical/biological events described herein, in the references cited herein, incorporated by reference for these endpoint assay targets, and other functions known to those of ordinary skill in the art.

Binding and/or activating compounds can also be screened by using chimeric neuritin proteins in which one or more regions, segments, sites, and the like, as disclosed herein. For example, a catalytic region can be used that interacts with a different peptide sequence specificity and/or affinity than the native neuritin. Accordingly, a different set of components is available as an end-point assay for activation. As a further alternative, the site of modification by an effector protein, for example phosphorylation, can be replaced with the site for a different effector protein. Activation can also be detected by a reporter gene containing an easily detectable coding region operably linked to a transcriptional regulatory sequence that is part of the native pathway in which the neuritin is involved.

The invention also provides antibodies that selectively bind to the neuritin and its variants and fragments. An antibody is considered to selectively bind, even if it also binds to other proteins that are not substantially homologous with the neuritin. These other proteins share homology with a fragment or domain of the neuritin. This conservation in specific regions gives rise to antibodies that bind to both proteins by virtue of the homologous sequence. In this case, it would be understood that antibody binding to the neuritin is still selective.

To generate antibodies, an isolated neuritin polypeptide is used as an immunogen to generate antibodies using standard techniques for polyclonal and monoclonal antibody preparation. Either the full-length protein or antigenic peptide fragment can be used.

Antibodies are preferably prepared from these regions or from discrete fragments in these regions. However, antibodies can be prepared from any region of the peptide as described herein. A preferred fragment produces an antibody that diminishes or completely prevents peptide hydrolysis or binding. Antibodies can be developed against the entire neuritin or domains of the neuritin as described herein. The antigenic peptide can comprise a contiguous sequence of at least 12, 14, 15, or 30 amino acid residues. In one embodiment, fragments correspond to regions that are located on the surface of the protein, e.g., hydrophilic regions. These fragments are not to be construed, however, as encompassing any fragments, which may be disclosed prior to the invention.

Antibodies can be polyclonal or monoclonal. An intact antibody, or a fragment thereof (e.g. Fab or F(ab').sub.2) can be used.

Detection can be facilitated by coupling (i.e., physically linking) the antibody to a detectable substance. Examples of detectable substances include various enzymes, prosthetic groups, fluorescent materials, luminescent materials, bioluminescent materials, and radioactive materials. Examples of suitable enzymes include horseradish peroxidase, alkaline phosphatase, .beta.-galactosidase, or acetylcholinesterase; examples of suitable prosthetic group complexes include streptavidin/biotin and avidinibiotin; examples of suitable fluorescent materials include umbelliferone, fluorescein, fluorescein isothiocyanate, rhodamine, dichlorotriazinylamine fluorescein, dansyl chloride or phycoerythrin; an example of a luminescent material includes luminol; examples of bioluminescent materials include luciferase, luciferin, and aequorin.

An appropriate immunogenic preparation can be derived from native, recombinantly expressed, or chemically synthesized peptides.

The antibodies can be used to assess neuritin expression in disease states such as in active stages of the disease or in an individual with a predisposition toward disease related to neuritin function. When a disorder is caused by an inappropriate tissue distribution, developmental expression, or level of expression of the neuritin protein, the antibody can be prepared against the normal neuritin protein. If a disorder is characterized by a specific mutation in the neuritin, antibodies specific for this mutant protein can be used to assay for the presence of the specific mutant neuritin. However, intracellularly-made antibodies ("intrabodies") are also encompassed, which would recognize intracellular neuritin peptide regions.

The antibodies can also be used to assess normal and aberrant subcellular localization of cells in the various tissues in an organism. Antibodies can be developed against the whole neuritin or portions of the neuritin.

The diagnostic uses can be applied, not only in genetic testing, but also in monitoring a treatment modality. Accordingly, where treatment is ultimately aimed at correcting neuritin expression level or the presence of aberrant neuritins and aberrant tissue distribution or developmental expression, antibodies directed against the neuritin or relevant fragments can be used to monitor therapeutic efficacy.

Antibodies accordingly can be used diagnostically to monitor protein levels in tissue as part of a clinical testing procedure, e.g., to, for example, determine the efficacy of a given treatment regimen.

The antibodies are also useful as diagnostic tools as an immunological marker for aberrant neuritin analyzed by electrophoretic mobility, isoelectric point, tryptic peptide digest, and other physical assays known to those in the art.

The antibodies are also useful for tissue typing. Thus, where a specific neuritin has been correlated with expression in a specific tissue, antibodies that are specific for this neuritin can be used to identify a tissue type.

The antibodies are also useful for inhibiting neuritin function.

These uses can also be applied in a therapeutic context in which treatment involves inhibiting neuritin function. An antibody can be used, for example, to block peptide binding. Antibodies can be prepared against specific fragments containing sites required for function or against intact neuritin associated with a cell.

Completely human antibodies are particularly desirable for therapeutic treatment of human patients. For an overview of this technology for producing human antibodies, see Lonberg et al. (1995) Int. Rev. Immunol. 13:65-93. For a detailed discussion of this technology for producing human antibodies and human monoclonal antibodies and protocols for producing such antibodies, e.g., U.S. Pat. No. 5,625,126; U.S. Pat. No. 5,633,425; U.S. Pat. No. 5,569,825; U.S. Pat. No. 5,661,016; and U.S. Pat. No. 5,545,806.

The invention also encompasses kits for using antibodies to detect the presence of an neuritin protein in a biological sample. The kit can comprise antibodies such as a labeled or labelable antibody and a compound or agent for detecting neuritin in a biological sample; means for determining the amount of neuritin in the sample; and means for comparing the amount of neuritin in the sample with a standard. The compound or agent can be packaged in a suitable container. The kit can further comprise instructions for using the kit to detect neuritin.

The invention also provides vectors containing the neuritin polynucleotides. The term "vector" refers to a vehicle, preferably a nucleic acid molecule that can transport the neuritin polynucleotides. When the vector is a nucleic acid molecule, the neuritin polynucleotides are covalently linked to the vector nucleic acid. With this aspect of the invention, the vector includes a plasmid, single or double stranded phage, a single or double stranded RNA or DNA viral vector, or artificial chromosome, such as a BAC, PAC, YAC, OR MAC.

A vector can be maintained in the host cell as an extrachromosomal element where it replicates and produces additional copies of the neuritin polynucleotides. Alternatively, the vector may integrate into the host cell genome and produce additional copies of the neuritin polynucleotides when the host cell replicates.

The invention provides vectors for the maintenance (cloning vectors) or vectors for expression (expression vectors) of the neuritin polynucleotides. The vectors can function in procaryotic or eukaryotic cells or in both (shuttle vectors).

Expression vectors contain cis-acting regulatory regions that are operably linked in the vector to the neuritin polynucleotides such that transcription of the polynucleotides is allowed in a host cell. The polynucleotides can be introduced into the host cell with a separate polynucleotide capable of affecting transcription. Thus, the second polynucleotide may provide a trans-acting factor interacting with the cis-regulatory control region to allow transcription of the neuritin polynucleotides from the vector.

Alternatively, a trans-acting factor may be supplied by the host cell. Finally, a trans-acting factor can be produced from the vector itself.

It is understood, however, that in some embodiments, transcription and/or translation of the neuritin polynucleotides can occur in a cell-free system.

The regulatory sequence to which the polynucleotides described herein can be operably linked include promoters for directing mRNA transcription. These include, but are not limited to, the left promoter from bacteriophage the lac, TRP, and TAC promoters from E. coli, the early and late promoters from SV40, the CMV immediate early promoter, the adenovirus early and late promoters, and retrovirus long-terminal repeats.

In addition to control regions that promote transcription, expression vectors may also include regions that modulate transcription, such as repressor binding sites and enhancers. Examples include the SV40 enhancer, the cytomegalovirus immediate early enhancer, polyoma enhancer, adenovirus enhancers, and retrovirus LTR enhancers.

In addition to containing sites for transcription initiation and control, expression vectors can also contain sequences necessary for transcription termination and, in the transcribed region a ribosome binding site for translation. Other regulatory control elements for expression include initiation and termination codons as well as polyadenylation signals. The person of ordinary skill in the art would be aware of the numerous regulatory sequences that are useful in expression vectors. Such regulatory sequences are described, for example, in Sambrook et al. (1989) Molecular Cloning: A Laboratory Manual 2nd. ed., Cold Spring Harbor Laboratory Press, Cold Spring Harbor, N.Y.).

A variety of expression vectors can be used to express an neuritin polynucleotide. Such vectors include chromosomal, episomal, and virus-derived vectors, for example vectors derived from bacterial plasmids, from bacteriophage, from yeast episomes, from yeast chromosomal elements, including yeast artificial chromosomes, from viruses such as baculoviruses, papovaviruses such as SV40, Vaccinia viruses, adenoviruses, poxviruses, pseudorabies viruses, and retroviruses. Vectors may also be derived from combinations of these sources such as those derived from plasmid and bacteriophage genetic elements, e.g. cosmids and phagemids. Appropriate cloning and expression vectors for prokaryotic and eukaryotic hosts are described in Sambrook et al. (1989) Molecular Cloning: A Laboratory Manual 2nd. ed., Cold Spring Harbor Laboratory Press, Cold Spring Harbor, N.Y.

The regulatory sequence may provide constitutive expression in one or more host cells (i.e. tissue specific) or may provide for inducible expression in one or more cell types such as by temperature, nutrient additive, or exogenous factor such as a hormone or other ligand. A variety of vectors providing for constitutive and inducible expression in prokaryotic and eukaryotic hosts are well known to those of ordinary skill in the art.

The neuritin polynucleotides can be inserted into the vector nucleic acid by well-known methodology. Generally, the DNA sequence that will ultimately be expressed is joined to an expression vector by cleaving the DNA sequence and the expression vector with one or more restriction enzymes and then ligating the fragments together. Procedures for restriction enzyme digestion and ligation are well known to those of ordinary skill in the art.

The vector containing the appropriate polynucleotide can be introduced into an appropriate host cell for propagation or expression using well-known techniques. Bacterial cells include, but are not limited to, E. coli, Streptomyces, and Salmonella typhimurium. Eukaryotic cells include, but are not limited to, yeast, insect cells such as Drosophila, animal cells such as COS and CHO cells, and plant cells.

As described herein, it may be desirable to express the polypeptide as a fusion protein. Accordingly, the invention provides fusion vectors that allow for the production of the neuritin polypeptides. Fusion vectors can increase the expression of a recombinant protein, increase the solubility of the recombinant protein, and aid in the purification of the protein by acting for example as a ligand for affinity purification. A proteolytic cleavage site may be introduced at the junction of the fusion moiety so that the desired polypeptide can ultimately be separated from the fusion moiety. Proteolytic enzymes include, but are not limited to, factor Xa, thrombin, and enterokinase. Typical fusion expression vectors include pGEX (Smith et al. (1988) Gene 67:31-40), pMAL (New England Biolabs, Beverly, Mass.) and pRIT5 (Pharmacia, Piscataway, N.J.) which fuse glutathione S-transferase (GST), maltose E binding protein, or protein A, respectively, to the target recombinant protein. Examples of suitable inducible non-fusion E. coli expression vectors include pTrc (Amann et al. (1988) Gene 69:301-315) and pET 11d (Studier et al. (1990) Gene Expression Technology: Methods in Enzymology 185:60-89).

Recombinant protein expression can be maximized in a host bacteria by providing a genetic background wherein the host cell has an impaired capacity to proteolytically cleave the recombinant protein. (Gottesman, S. (1990) Gene Expression Technology: Methods in Enzymology 185, Academic Press, San Diego, Calif. 119-128). Alternatively, the sequence of the polynucleotide of interest can be altered to provide preferential codon usage for a specific host cell, for example E. coli. (Wada et al. (1992) Nucleic Acids Res. 20:2111-2118).

The neuritin polynucleotides can also be expressed by expression vectors that are operative in yeast. Examples of vectors for expression in yeast e.g., S. cerevisiae include pYepSec1 (Baldari et al. (1987) EMBO J. 6:229-234), pMFa (Kurjan et al. (1982) Cell 30:933-943), pJRY88 (Schultz et al. (1987) Gene 54:113-123), and pYES2 (Invitrogen Corporation, San Diego, Calif.).

The neuritin polynucleotides can also be expressed in insect cells using, for example, baculovirus expression vectors. Baculovirus vectors available for expression of proteins in cultured insect cells (e.g., Sf 9 cells) include the pAc series (Smith et al. (1983) Mol. Cell Biol. 3:2156-2165) and the pVL series (Lucklow et al. (1989) Virology 170:31-39).

In certain embodiments of the invention, the polynucleotides described herein are expressed in mammalian cells using mammalian expression vectors. Examples of mammalian expression vectors include pCDM8 (Seed, B. (1987) Nature 329:840) and pMT2PC (Kaufman et al. (1987) EMBO J. 6:187-195).

The expression vectors listed herein are provided by way of example only of the well-known vectors available to those of ordinary skill in the art that would be useful to express the neuritin polynucleotides. The person of ordinary skill in the art would be aware of other vectors suitable for maintenance propagation or expression of the polynucleotides described herein. These are found for example in Sambrook et al. (1989) Molecular Cloning: A Laboratory Manual 2nd, ed., Cold Spring Harbor Laboratory, Cold Spring Harbor Laboratory Press, Cold Spring Harbor, N.Y.

The invention also encompasses vectors in which the nucleic acid sequences described herein are cloned into the vector in reverse orientation, but operably linked to a regulatory sequence that permits transcription of antisense RNA. Thus, an antisense transcript can be produced to all, or to a portion, of the polynucleotide sequences described herein, including both coding and non-coding regions. Expression of this antisense RNA is subject to each of the parameters described above in relation to expression of the sense RNA (regulatory sequences, constitutive or inducible expression, tissue-specific expression).

The invention also relates to recombinant host cells containing the vectors described herein. Host cells therefore include prokaryotic cells, lower eukaryotic cells such as yeast, other eukaryotic cells such as insect cells, and higher eukaryotic cells such as mammalian cells.

The recombinant host cells are prepared by introducing the vector constructs described herein into the cells by techniques readily available to the person of ordinary skill in the art. These include, but are not limited to, calcium phosphate transfection, DEAE-dextran-mediated transfection, cationic lipid-mediated transfection, electroporation, transduction, infection, lipofection, and other techniques such as those found in Sambrook et al. (Molecular Cloning: A Laboratory Manual, 2d ed., Cold Spring Harbor Laboratory, Cold Spring Harbor Laboratory Press, Cold Spring Harbor, N.Y.).

Host cells can contain more than one vector. Thus, different nucleotide sequences can be introduced on different vectors of the same cell. Similarly, the neuritin polynucleotides can be introduced either alone or with other polynucleotides that are not related to the neuritin polynucleotides such as those providing trans-acting factors for expression vectors. When more than one vector is introduced into a cell, the vectors can be introduced independently, co-introduced or joined to the neuritin polynucleotide vector.

In the case of bacteriophage and viral vectors, these can be introduced into cells as packaged or encapsulated virus by standard procedures for infection and transduction. Viral vectors can be replication-competent or replication-defective. In the case in which viral replication is defective, replication will occur in host cells providing functions that complement the defects.

Vectors generally include selectable markers that enable the selection of the subpopulation of cells that contain the recombinant vector constructs. The marker can be contained in the same vector that contains the polynucleotides described herein or may be on a separate vector. Markers include tetracycline or ampicillin-resistance genes for prokaryotic host cells and dihydrofolate reductase or neomycin resistance for eukaryotic host cells. However, any marker that provides selection for a phenotypic trait will be effective.

While the mature proteins can be produced in bacteria, yeast, mammalian cells, and other cells under the control of the appropriate regulatory sequences, cell-free transcription and translation systems can also be used to produce these proteins using RNA derived from the DNA constructs described herein.

Where secretion of the polypeptide is desired, appropriate secretion signals are incorporated into the vector. The signal sequence can be endogenous to the neuritin polypeptides or heterologous to these polypeptides.

Where the polypeptide is not secreted into the medium, the protein can be isolated from the host cell by standard disruption procedures, including freeze thaw, sonication, mechanical disruption, use of lysing agents and the like. The polypeptide can then be recovered and purified by well-known purification methods including ammonium sulfate precipitation, acid extraction, anion or cationic exchange chromatography, phosphocellulose chromatography, hydrophobic-interaction chromatography, affinity chromatography, hydroxylapatite chromatography, lectin chromatography, or high performance liquid chromatography.

It is also understood that depending upon the host cell in recombinant production of the polypeptides described herein, the polypeptides can have various glycosylation patterns, depending upon the cell, or maybe non-glycosylated as when produced in bacteria. In addition, the polypeptides may include an initial modified methionine in some cases as a result of a host-mediated process.

It is understood that "host cells" and "recombinant host cells" refer not only to the particular subject cell but also to the progeny or potential progeny of such a cell. Because certain modifications may occur in succeeding generations due to either mutation or environmental influences, such progeny may not, in fact, be identical to the parent cell, but are still included within the scope of the term as used herein.

The host cells expressing the polypeptides described herein, and particularly recombinant host cells, have a variety of uses. First, the cells are useful for producing neuritin proteins or polypeptides that can be further purified to produce desired amounts of neuritin protein or fragments. Thus, host cells containing expression vectors are useful for polypeptide production.

Host cells are also useful for conducting cell-based assays involving the neuritin or neuritin fragments. Thus, a recombinant host cell expressing a native neuritin is useful to assay for compounds that stimulate or inhibit neuritin function. This includes zinc or peptide binding, gene expression at the level of transcription or translation, and interaction with other cellular components.

Host cells are also useful for identifying neuritin mutants in which these functions are affected. If the mutants naturally occur and give rise to a pathology, host cells containing the mutations are useful to assay compounds that have a desired effect on the mutant neuritin (for example, stimulating or inhibiting function) which may not be indicated by their effect on the native neuritin.

Recombinant host cells are also useful for expressing the chimeric polypeptides described herein to assess compounds that activate or suppress activation by means of a heterologous domain, segment, site, and the like, as disclosed herein.

Further, mutant neuritins can be designed in which one or more of the various functions is engineered to be increased or decreased and used to augment or replace neuritin proteins in an individual. Thus, host cells can provide a therapeutic benefit by replacing an aberrant neuritin or providing an aberrant neuritin that provides a therapeutic result. In one embodiment, the cells provide neuritins that are abnormally active.

In another embodiment, the cells provide neuritins that are abnormally inactive. These neuritins can compete with endogenous neuritins in the individual.

In another embodiment, cells expressing neuritins that cannot be activated, are introduced into an individual in order to compete with endogenous neuritins for zinc or peptide. For example, in the case in which excessive zinc is part of a treatment modality, it may be necessary to effectively inactivate zinc at a specific point in treatment. Providing cells that compete for the molecule, but which cannot be affected by neuritin activation would be beneficial.

Homologously recombinant host cells can also be produced that allow the in situ alteration of endogenous neuritin polynucleotide sequences in a host cell genome. This technology is more fully described in WO 93/09222, WO 91/12650 and U.S. Pat. No. 5,641,670. Briefly, specific polynucleotide sequences corresponding to the neuritin polynucleotides or sequences proximal or distal to an neuritin gene are allowed to integrate into a host cell genome by homologous recombination where expression of the gene can be affected. In one embodiment, regulatory sequences are introduced that either increase or decrease expression of an endogenous sequence. Accordingly, an neuritin protein can be produced in a cell not normally producing it, or increased expression of neuritin protein can result in a cell normnally producing the protein at a specific level. Alternatively, the entire gene can be deleted. Still further, specific mutations can be introduced into any desired region of the gene to produce mutant neuritin proteins. Such mutations could be introduced, for example, into the specific regions disclosed herein.

In one embodiment, the host cell can be a fertilized oocyte or embryonic stem cell that can be used to produce a transgenic animal containing the altered neuritin gene. Alternatively, the host cell can be a stem cell or other early tissue precursor that gives rise to a specific subset of cells and can be used to produce transgenic tissues in an animal. See also Thomas et al., Cell 51:503 (1987) for a description of homologous recombination vectors. The vector is introduced into an embryonic stem cell line (e.g., by electroporation) and cells in which the introduced gene has homologously recombined with the endogenous neuritin gene is selected (see e.g., Li, E. et al. (1992) Cell 69:915). The selected cells are then injected into a blastocyst of an animal (e.g., a mouse) to form aggregation chimeras (see e.g., Bradley, A. in Teratocarcinomas and Embryonic Stem Cells: A Practical Approach, E. J. Robertson, ed. (IRL, Oxford, 1987) pp. 113-152). A chimeric embryo can then be implanted into a suitable pseudopregnant female foster animal and the embryo brought to term. Progeny harboring the homologously recombined DNA in their germ cells can be used to breed animals in which all cells of the animal contain the homologously recombined DNA by germline transmission of the transgene. Methods for constructing homologous recombination vectors and homologous recombinant animals are described further in Bradley, A. (1991) Current Opinion in Biotechnology 2:823-829 and in PCT International Publication Nos. WO 90/11354; WO 91/01140; and WO 93/04169.

The genetically engineered host cells can be used to produce non-human transgenic animals. A transgenic animal is preferably a mammal, for example a rodent, such as a rat or mouse, in which one or more of the cells of the animal include a transgene. A transgene is exogenous DNA which is integrated into the genome of a cell from which a transgenic animal develops and which remains in the genome of the mature animal in one or more cell types or tissues of the transgenic animal. These animals are useful for studying the function of an neuritin protein and identifying and evaluating modulators of neuritin protein activity.

Other examples of transgenic animals include non-human primates, sheep, dogs, cows, goats, chickens, and amphibians.

In one embodiment, a host cell is a fertilized oocyte or an embryonic stem cell into which neuritin polynucleotide sequences have been introduced.

A transgenic animal can be produced by introducing nucleic acid into the male pronuclei of a fertilized oocyte, e.g., by microinjection, retroviral infection, and allowing the oocyte to develop in a pseudopregnant female foster animal. Any of the neuritin nucleotide sequences can be introduced as a transgene into the genome of a non-human animal, such as a mouse.

Any of the regulatory or other sequences useful in expression vectors can form part of the transgenic sequence. This includes intronic sequences and polyadenylation signals, if not already included. A tissue-specific regulatory sequence(s) can be operably linked to the transgene to direct expression of the neuritin protein to particular cells.

Methods for generating transgenic animals via embryo manipulation and microinjection, particularly animals such as mice, have become conventional in the art and are described, for example, in U.S. Pat. Nos. 4,736,866 and 4,870,009, both by Leder et al., U.S. Pat. No. 4,873,191 by Wagner et al. and in Hogan, B., Manipulating the Mouse Embryo, (Cold Spring Harbor Laboratory Press, Cold Spring Harbor, N.Y., 1986). Similar methods are used for production of other transgenic animals. A transgenic founder animal can be identified based upon the presence of the transgene in its genome and/or expression of transgenic mRNA in tissues or cells of the animals. A transgenic founder animal can then be used to breed additional animals carrying the transgene. Moreover, transgenic animals carrying a transgene can further be bred to other transgenic animals carrying other transgenes. A transgenic animal also includes animals in which the entire animal or tissues in the animal have been produced using the homologously recombinant host cells described herein.

In another embodiment, transgenic non-human animals can be produced which contain selected systems, which allow for regulated expression of the transgene. One example of such a system is the cre/loxP recombinase system of bacteriophage P1. For a description of the cre/loxP recombinase system, see, e.g., Lakso et al. (1992) PNAS 89:6232-6236. Another example of a recombinase system is the FLP recombinase system of S. cerevisiae (O'Gorman et al. (1991) Science 251:1351-1355. If a cre/loxP recombinase system is used to regulate expression of the transgene, animals containing transgenes encoding both the Cre recombinase and a selected protein is required. Such animals can be provided through the construction of "double" transgenic animals, e.g., by mating two transgenic animals, one containing a transgene encoding a selected protein and the other containing a transgene encoding a recombinase.

Clones of the non-human transgenic animals described herein can also be produced according to the methods described in Wilmut et al. (1997) Nature 385:810-813 and PCT International Publication Nos. WO 97/07668 and WO 97/07669. In brief, a cell, e.g., a somatic cell, from the transgenic animal can be isolated and induced to exit the growth cycle and enter G.sub.o phase. The quiescent cell can then be fused, e.g., through the use of electrical pulses, to an enucleated oocyte from an animal of the same species from which the quiescent cell is isolated. The reconstructed oocyte is then cultured such that it develops to morula or blastocyst and then transferred to a pseudopregnant female foster animal. The offspring born of this female foster animal will be a clone of the animal from which the cell, e.g., the somatic cell, is isolated.

Transgenic animals containing recombinant cells that express the polypeptides described herein are useful to conduct the assays described herein in an in vivo context. Accordingly, the various physiological factors that are present in vivo and that could affect binding or activation, may not be evident from in vitro cell-free or cell-based assays. Accordingly, it is useful to provide non-human transgenic animals to assay in vivo neuritin function, including peptide interaction, the effect of specific mutant neuritins on neuritin function and peptide interaction, and the effect of chimeric neuritins. It is also possible to assess the effect of null mutations, that is mutations that substantially or completely eliminate one or more neuritin functions.

The neuritin nucleic acid molecules, protein, modulators of the protein, and antibodies (also referred to herein as "active compounds") can be incorporated into pharmaceutical compositions suitable for administration to a subject, e.g., a human. Moreover, the cells populations and modified cells identified herein can be incorporated into pharmaceutical compositions suitable for administration to a subject. Such compositions typically comprise the nucleic acid molecule, protein, modulator, or antibody and a pharmaceutically acceptable carrier.

The term "administer" is used in its broadest sense and includes any method of introducing the compositions of the present invention into a subject. This includes producing polypeptides or polynucleotides in vivo by in vivo transcription or translation of polynucleotides that have been exogenously introduced into a subject. Thus, polypeptides or nucleic acids produced in the subject from the exogenous compositions are encompassed in the term "administer."

As used herein the language "pharmaceutically acceptable carrier" is intended to include any and all solvents, dispersion media, coatings, antibacterial and antifungal agents, isotonic and absorption delaying agents, and the like, compatible with pharmaceutical administration. The use of such media and agents for pharmaceutically active substances is well known in the art. Except insofar as any conventional media or agent is incompatible with the active compound, such media can be used in the compositions of the invention. Supplementary active compounds can also be incorporated into the compositions. A pharmaceutical composition of the invention is formulated to be compatible with its intended route of administration. Examples of routes of administration include parenteral, e.g., intravenous, intradermal, subcutaneous, oral (e.g., inhalation), transdermal (topical), transmucosal, and rectal administration. Solutions or suspensions used for parenteral, intradermal, or subcutaneous application can include the following components: a sterile diluent such as water for injection, saline solution, fixed oils, polyethylene glycols, glycerine, propylene glycol or other synthetic solvents; antibacterial agents such as benzyl alcohol or methyl parabens; antioxidants such as ascorbic acid or sodium bisulfite; chelating agents such as ethylenediaminetetraacetic acid; buffers such as acetates, citrates or phosphates and agents for the adjustment of tonicity such as sodium chloride or dextrose. pH can be adjusted with acids or bases, such as hydrochloric acid or sodium hydroxide. The parenteral preparation can be enclosed in ampules, disposable syringes or multiple dose vials made of glass or plastic.

Pharmaceutical compositions suitable for injectable use include sterile aqueous solutions (where water soluble) or dispersions and sterile powders for the extemporaneous preparation of sterile injectable solutions or dispersion. For intravenous administration, suitable carriers include physiological saline, bacteriostatic water, Cremophor EL. (BASF, Parsippany, N.J.) or phosphate buffered saline (PBS). In all cases, the composition must be sterile and should be fluid to the extent that easy syringability exists. It must be stable under the conditions of manufacture and storage and must be preserved against the contaminating action of microorganisms such as bacteria and fungi. The carrier can be a solvent or dispersion medium containing, for example, water, ethanol, polyol (for example, glycerol, propylene glycol, and liquid polyethylene glycol, and the like), and suitable mixtures thereof. The proper fluidity can be maintained, for example, by the use of a coating such as lecithin, by the maintenance of the required particle size in the case of dispersion and by the use of surfactants. Prevention of the action of microorganisms can be achieved by various antibacterial and antifungal agents, for example, parabens, chlorobutanol, phenol, ascorbic acid, thimerosal, and the like. In many cases, it will be preferable to include isotonic agents, for example, sugars, polyalcohols such as mannitol, sorbitol, sodium chloride in the composition. Prolonged absorption of the injectable compositions can be brought about by including in the composition an agent which delays absorption, for example, aluminum monostearate and gelatin.

Sterile injectable solutions can be prepared by incorporating the active compound (e.g., an neuritin protein or anti- neuritin antibody) in the required amount in an appropriate solvent with one or a combination of ingredients enumerated above, as required, followed by filtered sterilization. Generally, dispersions are prepared by incorporating the active compound into a sterile vehicle which contains a basic dispersion medium and the required other ingredients from those enumerated above. In the case of sterile powders for the preparation of sterile injectable solutions, the preferred methods of preparation are vacuum drying and freeze-drying which yields a powder of the active ingredient plus any additional desired ingredient from a previously sterile-filtered solution thereof.

Oral compositions generally include an inert diluent or an edible carrier. They can be enclosed in gelatin capsules or compressed into tablets. For oral administration, the agent can be contained in enteric forms to survive the stomach or further coated or mixed to be released in a particular region of the GI tract by known methods. For the purpose of oral therapeutic administration, the active compound can be incorporated with excipients and used in the form of tablets, troches, or capsules. Oral compositions can also be prepared using a fluid carrier for use as a mouthwash, wherein the compound in the fluid carrier is applied orally and swished and expectorated or swallowed. Pharmaceutically compatible binding agents, and/or adjuvant materials can be included as part of the composition. The tablets, pills, capsules, troches and the like can contain any of the following ingredients, or compounds of a similar nature: a binder such as microcrystalline cellulose, gum tragacanth or gelatin; an excipient such as starch or lactose, a disintegrating agent such as alginic acid, Primogel, or corn starch; a lubricant such as magnesium stearate or Sterotes; a glidant such as colloidal silicon dioxide; a sweetening agent such as sucrose or saccharin; or a flavoring agent such as peppermint, methyl salicylate, or orange flavoring.

For administration by inhalation, the compounds are delivered in the form of an aerosol spray from pressured container or dispenser, which contains a suitable propellant, e.g., a gas such as carbon dioxide, or a nebulizer.

Systemic administration can also be by transmucosal or transdermal means. For transmucosal or transdermal administration, penetrants appropriate to the barrier to be permeated are used in the formulation. Such penetrants are generally known in the art, and include, for example, for transmucosal administration, detergents, bile salts, and fusidic acid derivatives. Transmucosal administration can be accomplished through the use of nasal sprays or suppositories. For transdermal administration, the active compounds are formulated into ointments, salves, gels, or creams as generally known in the art.

The compounds can also be prepared in the form of suppositories (e.g., with conventional suppository bases such as cocoa butter and other glycerides) or retention enemas for rectal delivery.

In one embodiment, the active compounds are prepared with carriers that will protect the compound against rapid elimination from the body, such as a controlled release formulation, including implants and microencapsulated delivery systems. Biodegradable, biocompatible polymers can be used, such as ethylene vinyl acetate, polyanhydrides, polyglycolic acid, collagen, polyorthoesters, and polylactic acid. Methods for preparation of such formulations will be apparent to those skilled in the art. The materials can also be obtained commercially from Alza Corporation and Nova Pharmaceuticals, Inc. Liposomal suspensions (including liposomes targeted to infected cells with monoclonal antibodies to viral antigens) can also be used as pharmaceutically acceptable carriers. These can be prepared according to methods known to those skilled in the art, for example, as described in U.S. Pat. No. 4,522,811.

It is especially advantageous to formulate oral or parenteral compositions in dosage unit form for ease of administration and uniformity of dosage. "Dosage unit form" as used herein refers to physically discrete units suited as unitary dosages for the subject to be treated; each unit containing a predetermined quantity of active compound calculated to produce the desired therapeutic effect in association with the required pharmaceutical carrier. The specification for the dosage unit forms of the invention are dictated by and directly dependent on the unique characteristics of the active compound and the particular therapeutic effect to be achieved, and the limitations inherent in the art of compounding such an active compound for the treatment of individuals.

The nucleic acid molecules of the invention can be inserted into vectors and used as gene therapy vectors. Gene therapy vectors can be delivered to a subject by, for example, intravenous injection, local administration (U.S. Pat. No. 5,328,470) or by stereotactic injection (see e.g., Chen et al. (1994) PNAS 91:3054-3057). The pharmaceutical preparation of the gene therapy vector can include the gene therapy vector in an acceptable diluent, or can comprise a slow release matrix in which the gene delivery vehicle is imbedded. Alternatively, where the complete gene delivery vector can be produced intact from recombinant cells, e.g. retroviral vectors, the pharmaceutical preparation can include one or more cells which produce the gene delivery system.

The pharmaceutical compositions can be included in a container, pack, or dispenser together with instructions for administration.

This invention may be embodied in many different forms and should not be construed as limited to the embodiments set forth herein; rather, these embodiments are provided so that this disclosure will fully convey the invention to those skilled in the art. Many modifications and other embodiments of the invention will come to mind in one skilled in the art to which this invention pertains having the benefit of the teachings presented in the foregoing description. Although specific terms are employed, they are used as in the art unless otherwise indicated.

### EXAMPLES

It should be appreciated that the invention should not be construed to be limited to the examples that are now described; rather, the invention should be construed to include any and all applications provided herein and all equivalent variations within the skill of the ordinary artisan.

### Anergy induction in C3-HA transgenic mice

We have been studying the mechanism of T cell tolerance induction to self-antigens and tumor antigens using the approach of adoptive transfer of TCR transgenic CD4 T cells specific for a model antigen - hemaglutinin (HA) - expressed either as a viral antigen, a self antigen or a tumor antigen. After immunization of mice with recombinant vaccinia-HA (Vac-HA, 5 x 10⁶ pfu IP), adoptively transferred HA-specific 6.5 CD4 T cells undergo a typical effector/memory response characterized by an expansion/contraction phase and development of memory markers. When removed from the adoptively transferred animal, these effector/memory cells are hyper-responsive to HA *in vitro* relative to naive 6.5 T cells.

To understand *in vivo* tolerance mechanisms, we generated a HA transgenic mouse by expressing HA as a transgene under control of the C3 promoter. These mice express HA in many epithelial compartments with the highest level of expression in pulmonary epithelial cells (Adler, 1998). Transfer of 6.5 T cells into C3HA mouse strains results in an expansion/contraction phase similar to the response of Vac-HA, however, the resultant T cells become anergic. This anergic phenotype is characterized by their inability to produce IL2 or IFN-γ or proliferate in response to cognate antigen *in vitro* (Fig. 1).

Anergy induction can be observed by 4-5 days post T cell transfer. However, the early activation stage between day 1 and day 4 is characterized by the acquisition of effector functions, such as IFN-γ production, that are very similar to T cells activated in response to infection with recombinant Vaccinia-HA (Huang, 2003). These activated cells capable of causing an autoimmune response in tissues that express high levels of the antigen. Thus, if <0.5 x 10⁶ 6.5 T cells are transferred IV into C3-HA mice, the mice survive and 6.5 cells become allergic (Fig. 2). However, iv transfer of 2.5 x 10⁶ 6.5 CD4 T cells into C3-HA mice induces a severe pneumonitis characterized by a significant pulmonary vasculitis component with mortality by 4 to 7 days, even as 6.5 T cells themselves are becoming anergic. This pulmonary vasculitis is characterized by massive accumulation of 6.5 cells in the lungs together with macrophages and CD8 T cells (Which also contribute to the lethal pneumonitis). Transfer of a smaller number of 6.5 CD4 cells can induce a transient pneumonitis in C3-HA mice but is not lethal (Fig. 2).

Similar to the C3-HA self-antigen anergy model, when 6.5 T cells are transferred to mice bearing HA-expressing tumors (either A20-HA lymphoma or RENCA-HA renal cell carcinoma) they differentiate into similar anergic state (Staveley-O'Carroll, 1998;Sotomayor, 2001). These comparative studies emphasize the capacity of established tumors to induce antigen specific T cell tolerance via cross-tolerance mechanisms involving re-presentation of antigens by bone-marrow derived APCs.

### Anergic 6.5 T cells exhibit Treg function

Lechler's group has demonstrated that *in vitro* generated anergic T cells can exhibit suppression activity toward naive T cell activation in 1994 (Lombardi, 1994). This prompt us to ask whether *in vivo* generated anergic 6.5 T cells have any regulatory activity *in vitro* and *in vivo.* When *in vivo* anergized 6.5 Treg cells are reisolated, they suppressed naive T cell activation in an *in vitro* suppression assay (Fig. 3A). Evidence that the "anergic" 6.5 CD4 cells demonstrate Treg function *in vivo* came when animals pretreated with a sub-lethal dose of 6.5 cells were subsequently challenged with a lethal dose of 6.5 T cells. We found that as few as 8,000 initially transferred 6.5 CD4 T cells could protect animals from subsequent challenge with the lethal dose of 2.5 x 10⁶ 6.5 CD4 T cells (Fig. 2). Reduced cell expansion was observed in the protected animal among subsequent transferred T cells (Fig. 3B). Since elimination of CD25+ T cells prior to the adoptive transfer did not eliminate the capacity of cells from first transfer to protect animals from a subsequent lethal challenge of 6.5 cells, it is most likely that the protection is due to the Treg phenotype acquired by the first batch of adoptively transferred cells (as opposed to the presence of the naturally occurring Treg cells among the adoptive transferred population). Further evidence that the anergic cells demonstrate Treg function comes from the finding that they inhibit that activation to cytotoxicity of HA-specific CD8 cells after infection with Vac-HA (data from *in vitro* and *in vivo* CTL assays not shown). These findings are highly compatible with the published findings of Von Boehmer and colleagues, who demonstrated that 6.5 CD4 cells rendered anergic after transfer into transgenic mice expressing HA in the B cell compartment could in fact, exhibit Treg function (Jooss, 2001).

### Gene expression profiling of anergic/Treg cells

In order to identify genes associated with the anergic/Treg phenotype in our *in vivo* system, we performed Affymetrix chip analysis on purified 6.5 cells after either adoptive transfer into naive recipients followed by Vac-HA immunization to generate effector/memory T cells or after transfer into C3-HA mice. Donor 6.5 cells were crossed onto a Thyl.1 background that allowed for a two step isolation and purification procedure after adoptive transfer. 6.5 cells were then enriched to roughly 95% purity by sorting for CD4+Thy 1.1+ cells. This protocol avoids the use of TCR-specific antibodies that could potentially alter TCR dependent gene expression patterns based on the antibody isolation procedures. RNA was isolated from cells at days 0-4 post-adoptive transfer for chip analysis. Genes that were selectively expressed in anergic/Treg populations relative to effector/memory populations were rank ordered according to their mean differential expression on days 1-4 (Fig. 4).

A surprisingly large number of genes were selectively activated in anergic/Treg populations relative to effector/memory populations even at early time points post adoptive transfer. Many of these genes represented ESTs with no known function. Among the genes that have been previously identified, the gene that was among the most differentially expressed between anergic/Treg populations relative to effector/memory populations was Neuritin. Figure 5 demonstrates the relative expression of Neuritin based on the Affymetrix chip analysis comparing day 0 to day 2, 3 and 4 after adoptive transfer.

The gene expression profiling approach was also used to analyze anergic T cells generated based on an *in vitro* T cell anergy model. T helper 1 type cell clone AE7 was used in the *in vitro* anergy model as originally described by Schwartz's group (Jenkins, 1987). Thus, if AE7 cells are stimulated with TCR signal only (anti-CD3) in the absence of any costimulation, these cells would become anergic for an extended period of time. Even after resting for 10 days in the absence of IL2, cells will still be unresponsive upon restimulation and fail to produce any IL2. On the other hand, if the cells are provided with both TCR and costimulatory signals (anti-CD3+anti-CD28), AE7 cells show good proliferation and IL2 production upon restimulation. Interestingly, anergic AE7 cells can also suppress naive T cell activation (data not shown). Gene expression profile analysis was carried out on AE7 cells activated under anergy condition for various times. RNA was isolated from cells 2 hr, 4 hr and 6 hr post anergy induction and subjected to Affymetrix chip assay. Consistent with data from *in vivo* generated anergic Treg cells, Neuritin also showed more than 5 fold increased expression in cells from the anergy condition comparing to the resting AE7 cells (Fig. 5) and activated AE7 cells.

### Regulatory T cells express highest level of neuritin in the immune system.

Neuritin was originally identified during screening for hippocampal genes that are induced by neural activity (Naeve, 1997). Besides high expression levels in the brain, we also detected Neuritin expression in the kidney by western blot (data not shown). Neuritin expression is induced by neuronal activity and by activity-regulated neurotrophins BDNF and NT-3. Sequence analysis predicts an extracellular molecule, anchored to the membrane by a glycosylphosphatidylinositol (GPI) linkage, and no significant sequence or motif homology to any other protein has been found (Naeve, 1997). The protein sequences of Neuritin from human, rat and mouse are 100% conserved in the extracellular region. In neural systems, ectopic expression of Neuritin in neurons promotes dendritic arbor growth in neighboring cells through an intercellular signaling mechanism that requires its GPI link (Nedivi, 1998 ;Naeve, 1997). Recently, a soluble form of neuritin has been identified in the brain and shown to protect neurons from apoptosis (Putz, 2005).

Neuritin is a new molecule in the immunology field. To understand the function of neuritin in immune response, we first surveyed neuritin expression among different cell types. We compared neuritin expression in CD4+CD441ow naive T cell, CD4+CD44hiCD25+, CD4+CD44hiCD25-, CD8+CD441ow, CD8+CD44hi, B220+CD19+ cells, NK cells, CDlIChiB220- bmDCs, and CD11C1owB220+ plasmacytoid DCs. As shown in Fig. 6 of qRT-PCR analysis, Neuritin expressed the highest in CD4+CD25+ regulatory T cells. CD4+CD44hiCD25- and CD8+CD44hi antigen experienced T cells also expressed relatively high level of neuritin mRNA. Low level expression is detected in CD8+CD441ow T cells, B cells and bmDCs. It should be pointed out that in the qRT-PCR reaction, the amplification in naive T cells is the same as the negative control; thus, neuritin does not express in naive T cells. The expression in NK and pDC are also close to the baseline. Although we only carried out qRT-PCR on freshly sorted CDlIChiB220- bmDCs, we have examined neuritin expression in bmDCs activated under 8 different conditions and 4 different time points in our DNA microarray data bank and we did not see any significant changes, and the signals are all very low.

### Neuritin is specifically expressed in the Treg cells and its expression is regulated by IL2 and TCR signal.

Natural CD4+CD25+ Treg cells represent a heterogeneous population. They can be separated into two subpopulations: the CD4CD25+CD62hi - thought to be quiescent cells and CD4CD25+CD621ow cells that have various activation markers (Fisson, 2003). Although both populations had been shown to be equally anergic and suppressive upon polyclonal stimulation in vitro (Kuniyasu, 2000;Thornton, 2000; Szanya, 2002), CD4CD25+CD62hi cells showed longer lifespan and suppressor activity in vivo (Fisson, 2003; Taylor, 2004). It has been proposed that CD4CD25+CD621ow cells are activated effector Treg cells (Fisson, 2003; Huehn, 2004). We further examined neuritin expression pattern in sorted natural Treg subpopulations. While both CD4CD25+CD62hi and CD62low cells express neuritin, CD4CD25+CD621ow cells have much higher neuritin expression (Fig. 7A). To examine whether neuritin expression can be induced in the quiescent CD4CD25+CD62hi cells, we sorted these cells from naive Balb/C mice and treated with IL2, IL2+anti-CD3, CD3, CD3+CD28 and CD28 alone or without any treatment(rest). After overnight treatment (20hr), neuritin expression was induced by the addition of IL2 and 1L2+ani-CD3 (Fig. 7B). 1L-2 is essential to Treg cell survival, expansion and function in vivo (Malek TR, 2004). This result suggests neuritin maybe an effector gene for IL-2 mediated function in Treg cells.

### Neurtin is highly expressed in anergized T cells.

Genechip analysis indicate that neuritin is expressed in anergized T cells (Fig.5). This result is further confirmed by quantitative RT-PCR (qRT-PCR) performed on samples from *in vivo* anergized cells isolated at different times over a one month period after adoptive transfer of 6.5 CD4 cells into C3HA hosts (Fig. 8A). These data reaffirm that Neuritin expression is increased in anergized T cells over an extended period of time *in vivo.* In contrast, cells activated under memory inducing conditions (6.5 CD4 cells transferred into nontransgenic B10.D2 hosts and activated by Vaccinia virus encoding HA antigen) did not show increased neuritin expression, suggesting increased neuritin expression is specific to anergized T cells. The overexpression of Neuritin in the AE7 anergy system was further confirmed both at the RNA and protein level (Fig. 8 B&C).

To further investigate whether Neuritin shows a similar expression pattern in tumor anergized cells, A20 tumor anergizing system was used ( Staveley-O'Carroll, 1998). A20 is a BALB/c B cell lymphoma that behaves similarly *in vivo* to many forms of human B cell lymphoma. When injected into BALB/c mice, this tumor infiltrates the mesenteric lymph nodes, spleen and liver, and it can be found in the bone marrow and peripheral blood. A20 cells constitutively expressing HA antigen was used in the tumor anergizing experiment. Ten days after transfer of A20HA cells into BALB/c mice, HA antigen specific 6.5 T cells were adoptively transferred into the tumor-bearing host. 21 days after adoptive transfer, 6.5 T cells were isolated from the host and RNA samples were prepared. Neuritin expression level was compared among anergizing conditions (i.e. 6.5 T cell adoptive transferred to the A20HA host), naive conditions (i.e. 6.5 T cells transferred to A20 wild type host without HA antigen) and activation conditions (i.e. Vac HA activated 6.5 T cells) (Fig. 8D). Neuritin only showed increased expression under tumor anergizing conditions.

### Generation of T cell specific Neuritin overexpression transgenic mice.

In order to further understand the role of neuritin in T cell development and *in vivo* immune responses, we generated T cell specific Neuritin transgenic mice. This mouse is a powerful adjunct to the Neuritin Knock Out mice (described below) to compare in parallel immune responses in gain-of-function and loss-of-function mice. The improved human CD2 promoter can direct gene expression in virtually all single positive CD4+CD8- and CD4-CD8+ mature T lymphocytes in transgenic mice and the expression is linearly proportional to the transgene copy numbers (Zhumabekov, 1995). Neuritin was placed directly under the hCD2 promoter (Fig. 9A). The hCD2 neuritin expression cassette was purified and injected according to the standard procedures for generating transgenic mice. An exemplary transgene positive line is shown in (Fig. 9B). Comparison of neuritin expression between transgene positive vs. negative littermates clearly indicates that neuritin is over expressed in T cells from the transgene positive mice (Fig. 9C&D).

Analysis of the transgenic line indicates normal thymus development, with comparable percentage of CD4+, CD8+ and CD4+CD8+ cells (data not shown). Staining of peripheral lymphoid organs show comparable CD4+, CD8+, B220+, Gr-1+, DX5+ and CD11c+ cell populations. To examine T cell activation, CD4 and CD8 cells were purified and stimulated with plate-bound anti-CD3+CD28 and we have observed comparable T cell proliferation (data not shown). We've also observed similar number of CD4+CD25+ natural Treg cells with comparable suppression activity in vitro (data not shown).

### CD2N transgenic T cells show reduced suppression in vivo due to loss of Adoptive Transfer (ADT) CD2N+ cells.

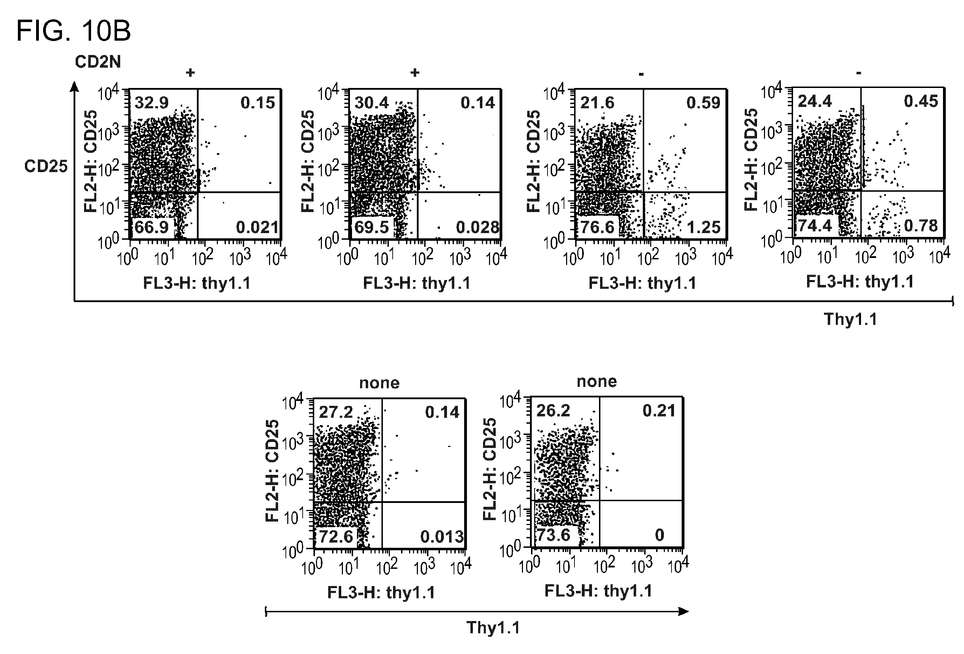

CD2N+ mice were backcrossed to B10.D2 background and with 6.5 TCR transgenic mice. To study the role of neuritin in anergy induction and its potential function in suppression, we adoptive transferred (ADT) 6.5 TCR+ ethyl.1+ CD2N+ or CD2N- total splenocytes into C3-HA thy1.2+ HA expressing hosts. 18 days after the ADT, CFSE labeled wt 6.5 T cells were ADT again into those and naive C3-HA hosts (without any pretransfer). Suppression of wt 6.5 T cell expansion by pretransferred CD2N+/- 6.5 cells were analyzed 3 days post second ADT. To our surprise, while CD2N- 6.5 T cells suppressed the proliferation of wt 6.5 T cells comparing to ADT into naive hosts, the suppression by CD2N+ 6.5 T cells were lost (Fig. 10A). At the mean time, the number of CD2N+ 6.5 cells in the C3-HA hosts was greatly reduced comparing to the CD2N- 6.5 cells (Fig. 10B).

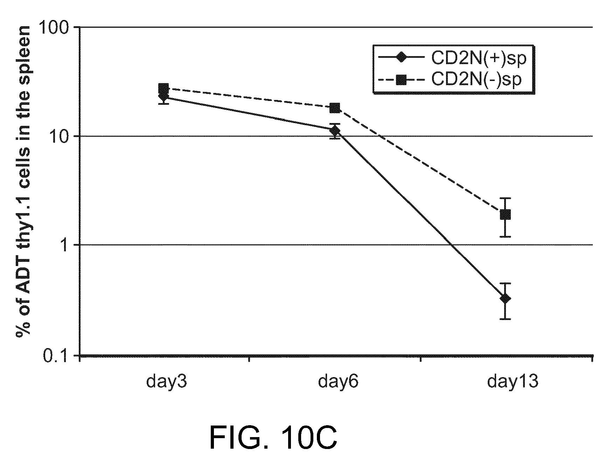

The loss of suppression and cells upon ADT into C3-HA hosts could be due to defective proliferation and/or defective induction of anergy in CD2N+ 6.5 cells. The proliferation of CD2N+ 6.5 cells were examined in vitro by observing CFSE dilution upon stimulation by HA peptide and in vivo by ADT into C3-HA hosts and examine CFSE dilution 3 days post ADT. Comparable CFSE dilution was observed among CD2N+/- 6.5 cells in vitro and in vivo (data not shown). Similar percentage of IL2 and IFNg positive cells were also observed among CD2N+/- activated cells in vitro and in vivo (data not shown). Furthermore, when analyzing CD2N+/- 6.5 cells ADT into C3-HA host at 3, 6, and 14 days post ADT, significant reduction of CD2N+ 6.5 cells only observed at later time point, while the number of cells were comparable day3 post ADT (Fig.10C). Taken together, these results indicate that overexpression of neuritin in CD4 T cells results in diminished persistence of self-Ag specific cells in vivo. The loss of CD2N+ cells overtime in C3-HA hosts is not due to defective activation and proliferation of neuritin expressing cells.

### Soluble neuritin mediates enhance antigen-specific T cell loss in self-antigen expressing hosts.

Previous section demonstrates that overexpression of neuritin in 6.5 T cells results in cell loss in self-antigen expressing C3-HA hosts. It is not clear whether this effect is intrinsic to neuritin expressing cells or neuritin can mediate it through a trans- mechanism. To investigate this, we cotransferred wt thy1.1+thy1.2- 6.5 T cells with CD2N+/- thy1.1+thy1.2+6.5 T cells into C3-HA hosts and observed the wt 6.5 cells over time. Although similar number of wt 6.5 cells was observed when cotransferred with CD2N+/- thy1.1+thy1.2+6.5 day3 post ADT, the number of wt 6.5 cells was greatly reduced on day 14 post ADT with CD2N+ cells (Fig.11A). This result suggests that the cell loss we observed in CD2N+ self-Ag specific T cells is not due to any developmental defect caused by neuritin overexpression. Neuritin may function in a soluble form, affecting the persistence of T cells.

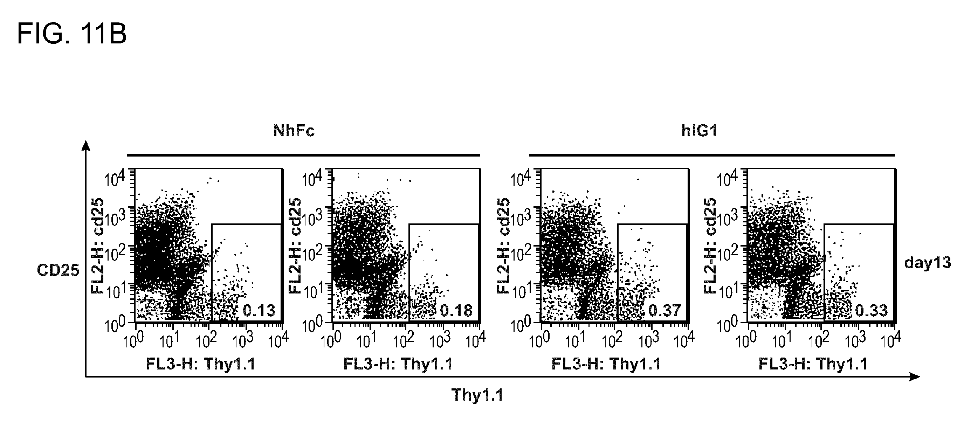

We further investigated whether soluble neuritin can mediate the trans-effect on cell loss in vivo. Wt 6.5 T cells were transferred into C3-HA hosts. Mice were injected i.p. with 200ug of either soluble neuritin human-Fc fusion protein (NhFc) or human IgG (hIgG) control at the time of cell transfer and every other day post ADT. The persistence of 6.5 cells in C3-HA hosts was examined 13 days post ADT. Treatment of NhFc results in enhanced loss of self-antigen specific wt cells (Fig.11B). Thus, soluble form of neuritin can mediate the effect on antigen specific T cell loss in self-antigen expressing hosts.

### Precipitated Foxp3+ Treg cell loss upon encounter antigen.

There are about 5% of 6.5+ CD4+CD25+ foxp3+ HA antigen-specific natural Treg cells in wt 6.5 TCR transgenic mice. Anergic CD4+CD25+ Treg cells express the Foxp3 transcription factor, which is a specific marker for cells that possess the Treg suppressive function. Similar percentages of 6.5+ CD4+CD25+foxp3+ natural Treg cells were observed among CD2N+ and CD2N- mice. Upon transfer of wt 6.5+ T cells into C3HA hosts, both CD4+CD25-naïve cells and CD4+CD25+ Treg cells expand (data not shown). Neuritin has been reported to support neuron survival and dendrite growth. It is possible that activation induced overexpression of neuritin mediate enhanced expansion or activity of foxp3+ Treg cells. Increased number and/or activity of Treg cells may in turn suppress foxp3- cell expansion and survival, leading to precipitated cell loss among ADT cells. To explore this possibility, we monitored the ratio of foxp3+ cells in total 6.5 + T cells from CD2N+/- mice after ADT into C3-HA host. If neuritin mediate enhanced expansion of natural Treg cells, cells from CD2N+ mice would show increased ratio of foxp3+ vs. foxp3- cells. On the contrary, foxp3+ cells from CD2N+ mice disappear more rapidly comparing to cells from the littermate control (Fig.12). Since similar percentages of Treg cells were found in CD2N+ /mice, the loss of Treg cells seems specific to regulatory T cell activation and expansion condition. This result suggests that enhanced neuritin expression upon Treg activation may serve to curb the exuberant expansion of Treg cells.

### Exacerbated disease in CD2N+ mice upon EAE induction

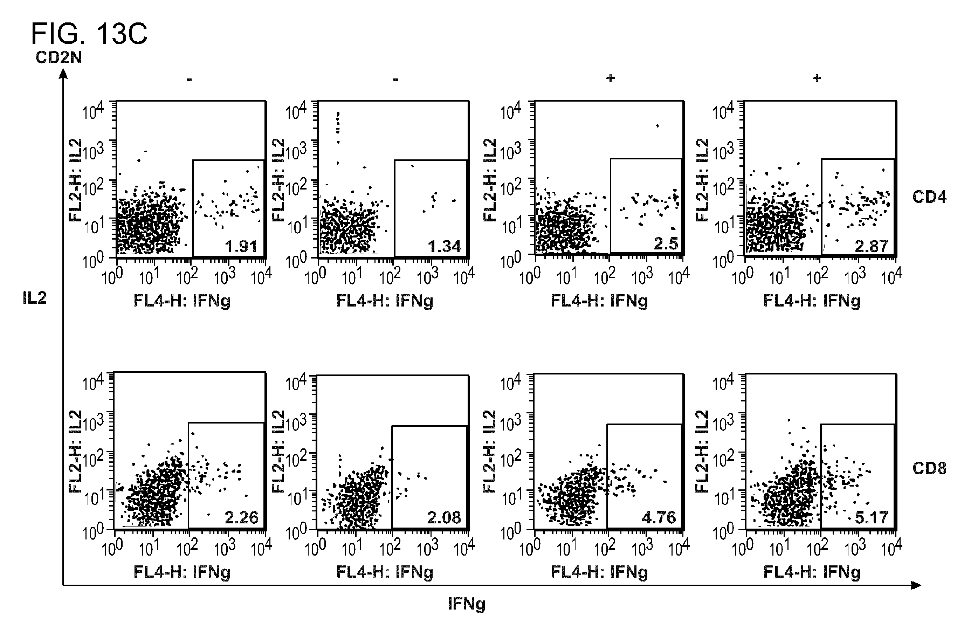

Disrupting the ratio of antigen specific Treg and effector cells can lead to pathologic conditions (Kohm, 2002). We choose to use myelin-oligodendrocyte-glycoprotein MOG (35-55) peptide mediated EAE autoimmune disease model to examine the impact of neuritin overexpression in the development of autoimmune disease. CD2N+ mice were backcrossed to C57/BL6 background and subjected to MOG mediated EAE induction. Upon EAE induction, CD2N+ mice showed earlier and more severe disease comparing to the CD2N-mice (Fig.13A). Upon restimulation of cells from draining lymph node and spleen with MOG peptide, increased IL17 secretion was detected by ELISA and more IFNg+ cells were detected by intracellular cytokine staining (Fig. 13 B&C). Moreover, increased number of CD4+ cells was recovered in CNS. Thus, overexpression of neuritin results in more severe disease in EAE.

### Neuritin-Fc can bind to activated DCs:

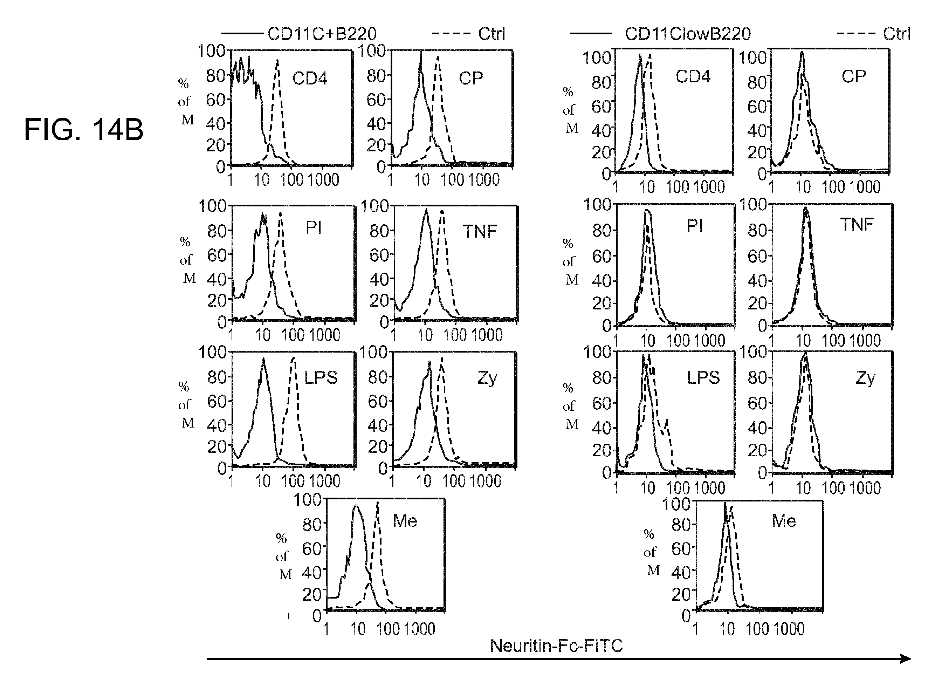

Neuritin is a GPI-anchored cell surface protein. It is therefore likely that it exerts its function through a receptor on other cells. To investigate the binding partner of Neuritin, a Neuritin-Fc (N-Fc) fusion protein was generated. The GPI-anchor of Neuritin was replaced with human IgG1 Fc portion and the recombinant protein was purified using a protein G column. The purified N-Fc was conjugated with FITC following manufacture's protocol and was used to stain cells from lymphoid tissues. No significant staining was observed in lymph node and spleen. In bone marrow derived dendritic cells (DCs), the level of N-Fc staining is quite low. However, upon activation with zymosan, a toll like receptor 2 ligand from yeast membrane, there is a dramatic increase in N-Fc staining (Fig. 14A). Similarly, fresh spleenic DCs do not stain with N-Fc. After *in vitro* culture for 24hrs, there is increased staining (Fig. 14B). Thus, Neuritin is capable of interacting with receptor expressed by activated DCs.

### Neuritin affects DC activation

Treg cells can actively suppress T cell responses via the antigen-presenting cell (Taams, 1998; Vendetti, 2000; Frasca, 2002; Cederbom, 2000; Misra, 2004;Min, 2003). Using T cell clones rendered anergic by stimulation in the presence of TCR signal alone, Frasca et al demonstrated that suppression caused by the anergic T cells requires the presence of antigen presenting cell such as DCs. Anergic cells can down regulate the costimulatory molecule on DCs in a contact dependent manner (Frasca, 2002). Studies in CD4+CD25+ cells also show that natural Treg cells can down regulate costimulatory molecule and antigen-presenting function of DCs in a cell contact dependent manner (Cederbom, 2000; Misra, 2004). The molecular mechanism for these observations is not clear. As Treg expressed cell surface molecule capable of binding to DCs, we asked whether Neuritin plays any role in DC maturation and antigen presentation function. Soluble Neuritin-Fc was used to to treat bone marrow derived DCs. Supernatant from Neuritin-Fc treated cells were measured for cytokine secretion. As shown in Fig. 15, reduced secretion of IL12p40 were observed in Neuritin-Fc treated cells.

To understand the function of Neuritin engagement on DCs when presented on T cells, retroviral vector carrying Neuritin was used to infect activated 6.5 CD4 T cells. Infected cells marked by GFP were sorted out and used in coculture with day7 bone-marrow derived DCs pulsed with HA peptide antigen. Supernatant was collected 24hr after coculture and examined for IL12 production. Consistent with the observation from Neuritin-Fc treated DCs, there was significant reduction in IL12 secretion in DCs cocultured with Neuritin expressing T cells (Fig. 16 A&B). DCs from the coculture were further used to present HA class I antigen to HA-specific clone 4 TCR transgenic CD8 T cells. Reduced CD8 cell proliferation was observed in antigen presented by DCs precultured with Neuritin-expressing T cells (Fig. 16C).

It has been reported that the DCs used to successfully treat diabetes in NOD mice were defective in IL12 production (Menges, 2002). Inhibition of IL12 production by DCs was associated with increased IL10 production and became poor allostimulators in mixed lymphocyte reaction (Hill, 2003). Other studies also suggest the possibility of protective DCs with a semimature phenotype characterized with high level of MHC and costimulatory molecule but low inflammatory cytokine such as IL12 (Lutz, 2002). Neuritin treated DCs showed similar phenotype to the semimature DCs.

### Neuritin specific monoclonal antibody identifies soluble and cell associated neuritin.

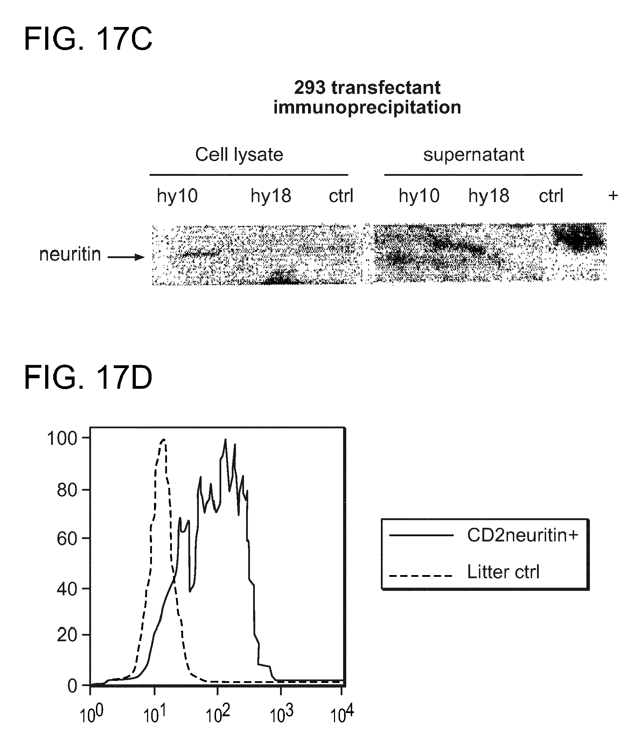

We have shown Neuritin expression is highly specific to T cells with Treg activity. Because of the 100% homology between murine and human Neuritin, monoclonal antibodies against Neuritin would be very useful to mark and study Treg cells in mice and humans. More over, the antibody may also be useful to modulate Neuritin activity in vivo. To generate neuritin-specific antibodies, three mice were immunized with neuritin mouse Fc fusion protein (neuritin-mFc). After 2 booster immunizations, the serum titer against neuritin was tested. The splenocytes from the mouse with the highest serum titer against neuritin was used to generate hybridomas. Supernatants from 150 hybridoma clones were initially tested by ELISA against neuritin-hFc protein. Sixteen clones tested positive by ELISA and were further tested for cell surface staining (Fig. 17A). 293 cells were transfected with a retroviral neuritin expression vector, which can mark the transfected cells with gfp. Two out of 16 clones showed positive staining on neuritin transfected 293 cells (Fig. 17B). The 1D6 antibody was also used to test whether it can immunoprecipitate neuritin from full-length or soluble neuritin transfected 293 cell lysate and supernatant. As shown in Fig. 17c, 1D6 can indeed immunoprecipitate neuritin from cell lysates as well as the supernatant of transfectants. To further test neuritin staining on primary T cells, supernatants from the two positive clones were used to stain thymocytes from CD2N+ transgenic mice and the littermate control. 200u1 of supernatant was incubated with I x 10⁶ thymocytes followed by anti-mouse-IgG-PE staining. The CD4+CD8- single positive T cells indeed stained positive with 1 D6 clone supernatant in CD2N+ transgenic mice comparing to the litter control (Fig. 17D). Clone 1A9, does not recognize neuritin expressed on primary T cells.

To examine endogenous neuritin surface expression, we labeled 1D6 antibody with biotin and stained for neuritin on lymph node cells and splenocytes from both CD2N+ and CD2N- mice. We have observed weak staining on CD4+CD44hiCD25- cells and CD4+CD44hiCD25+ cells in CD2N- mice (Fig. 18A), while the shift in CD4+CD25+CD44low cells was very small and hard to detect. Upon comparison to surface staining in subsets of CD4 cells from CD2N+ mice, we consistently observed that the surface staining of neuritin on CD4+CD25+ cells and CD4+CD44hiCD25- cells are lower than CD4+CD25-CD44low naive cells (Fig. 18B). Upon TCR stimulation of CD4 cells, we also observed decreased surface neuritin staining among activated but not naive transgenic T cells (data not shown). These observations strongly suggest the possibility of neuritin release from cell surface of Treg cells or in activated T cells. This result is also consistent with the functional role of soluble neuritin *in vivo* as described above.

We further used the two antibody clones against neuritin to examine endogenous soluble and membrane bound neuritin expression. These two antibody clones were used in the sandwich ELISA assy. C57/BL6 mice brain and kidney, the two organs identified to express neuritin protein by western blotting, were used to test the existence of soluble vs. cell associated neuritin. Brain and kidney tissue were grinded into cell suspension. Supernatant from tissue grinding and protein lysates from the cell pellet were tested for neuritin protein by sandwich ELISA using 1A9 and 1 D6 clones. As shown in Fig.l9, both soluble and cell associated neuritin were detected in the brain sample, whereas only cell associated neuritin was detected in the kidney. Thus, this assay confirms the existence of soluble neuritin in vivo. This assay will be used to assay differential neuritin forms in the immune system.

### Incorporation by Reference

The contents of all references, patents, pending patent applications and published patents, cited throughout this application are hereby expressly incorporated by reference.

### Equivalents

Those skilled in the art will recognize, or be able to ascertain using no more than routine experimentation, many equivalents to the specific embodiments of the invention described herein. Such equivalents are intended to be encompassed by the following claims.

### References

Adler, A.J., et al., CD4+ T cell tolerance to parenchymal self-antigens requires presentation by bone marrow-derived antigen presenting cells. J Exp Med, 1998. 187(10): p. 1555-64
Cantallops, I., K. Haas, and H.T. Cline, Postsynaptic CPG15 promotes synaptic maturation and presynaptic axon arbor elaboration in vivo. Nat Neurosci, 2000. 3(10): p. 1004-11.
Cederbom, L., H. Hall, and F. Ivars, CD4+CD25+ regulatory T cells down-regulate costimulatory molecules on antigen presenting cells. Eur J Immunol, 2000. 30(6): p. 1538-43.
Dustin, M.L. and D.R. Colman, Neural and immunological synaptic relations. Science, 2002. 298(5594): p. 785-9.
Fisson, S., G. Darrasse-Jeze, E. Litvinova, F. Septier, D. Klatzmann, R. Liblau, and B. L. Salomon. 2003. Continuous activation of autoreactive CD4+ CD25+ regulatory T cells in the steady state. J Exp Med 198: 737.
Frasca, L., et al., Human anergic CD4+ T cells can act as suppressor cells by affecting autologous dendritic cell conditioning and survival. J Immunol, 2002. 168(3): p. 1060-8.
Hill, J.A., et al., Immune modulation by silencing IL-12 production in dendritic cells using small interfering RNA. J Immunol, 2003. 171(2): p. 691-6.
Huang, C.T., et al., CD4+ T cells pass through an effector phase during the process of in vivo tolerance induction. J Immunol, 2003. 170(8): p. 3945-53.
Huehn, J., K. Siegmund, J. C. Lehmann, C. Siewert, U. Haubold, M. Feuerer, G. F. Debes, J. Lauber, O. Frey, G. K. Przybylski, U. Niesner, M. de la Rosa, C. A. Schmidt, R. Brauer, J. Buer, A. Scheffold, and A. Hamann. 2004. Developmental stage, phenotype, and migration distinguish naive- and effector/memory-like CD4+ regulatory T cells. J Exp Med 199:303.
Jenkins, M.K. and R.H. Schwartz, Antigen presentation by chemically modified splenocytes induces antigen-specific T cell unresponsiveness in vitro and in vivo. J Exp Med, 1987. 165(2): p. 302-19.
Jooss, K., et al., Regulatory function of in vivo anergized CD4(+) T cells. Proc Natl Acad Sci USA, 2001. 98(15): p. 8738-43.
Joyner, A., Gene Targeting. 2000.
Khan, A.A., et al., Physiological regulation of the immunological synapse by agrin. Science, 2001. 292(5522): p. 1681-6.
Kohm, A. P., P. A. Carpentier, H. A. Anger, and S. D. Miller. 2002. Cutting edge: CD4+CD25+ regulatory T cells suppress antigen-specific autoreactive immune responses and central nervous system inflammation during active experimental autoimmune encephalomyelitis. J Immunol 169:4712.
Kuniyasu, Y., T. Takahashi, M. Itoh, J. Shimizu, G. Toda, and S. Sakaguchi. 2000. Naturally anergic and suppressive CD25(+)CD4(+) T cells as a functionally and phenotypically distinct immunoregulatory T cell subpopulation. Int Immunol 12:1145.
Lamb, J. R., B. J. Skidmore, N. Green, J. M. Chiller, and M. Feldmann. 1983. Induction of tolerance in influenza virus-immune T lymphocyte clones with synthetic peptides of influenza hemagglutinin. J Exp Med 157:1434.
Lee, P.P., et al., A critical role for Dnmtl and DNA methylation in T cell development, function, and survival. Immunity, 2001. 15(5): p. 763-74.
Lombardi, G., et al., Anergic T cells as suppressor cells in vitro. Science, 1994. 264(5165): p. 1587-9.
Lutz, M.B. and G. Schuler, Immature, semi-mature and fully mature dendritic cells: which signals induce tolerance or immunity? Trends Immunol, 2002. 23(9): p. 445-9.
Malek TR, B. A. 2004. Tolerance, not immunity, crucially depends on IL2. Nat Rev Immunol 4:665.
Menges, M., et al., Repetitive injections of dendritic cells matured with tumor necrosis factor alpha induce antigen-specific protection of mice from autoimmunity. J Exp Med, 2002. 195(1): p. 15-21.
Min, W.P., et al., Inhibitory feedback loop between tolerogenic dendritic cells and regulatory T cells in transplant tolerance. J Immunol, 2003. 170(3): p. 1304-12.
Misra, N., et al., Cutting Edge: Human CD4+CD25+ T cells restrain the maturation and antigen presenting function of dendritic cells. J Immunol, 2004. 172(8): p. 4676-80.
Naeve, G. S., M. Ramakrishnan, R. Kramer, D. Hevroni, Y. Citri, and L. E. Theill. 1997. Neuritin: a gene induced by neural activity and neurotrophins that promotes neuritogenesis. Proc Natl Acad Sci U S A 94:2648.
Nedivi, E., G.Y. Wu, and H.T. Cline, Promotion of dendritic growth by CPG15, an activity-induced signaling molecule. Science, 1998. 281(5384): p. 1863-6.
Putz, U., C. Harwell, and E. Nedivi. 2005. Soluble CPG15 expressed during early development rescues cortical progenitors from apoptosis. Nat Neurosci 8:322.
Sledz, C.A., et al., Activation of the interferon system by short-interfering RNAs. Nat Cell Biol, 2003. 5(9): p. 834-9.
Sotomayor, E.M., et al., Cross presentation of tumor antigens by bone marrow-derived antigen presenting cells is the dominant mechanism in the induction of T-cell tolerance during B-cell lymphoma progression. Blood, 2001. 98(4): p. 1070 7.
Staveley-O'Carroll, K., et al., Induction of antigen-specific T cell anergy: An early event in the course of tumor progression. Proc Natl Acad Sci USA, 1998. 95(3): p. 1178-83.
Szanya, V., J. Ermann, C. Taylor, C. Holness, and C. G. Fathman. 2002. The subpopulation of CD4+CD25+ splenocytes that delays adoptive transfer of diabetes expresses L-selectin and high levels of CCR7. J Immunol 169:2461.
Taams, L.S., et al., Anergic T cells actively suppress T cell responses via the antigen presenting cell. Eur J Immunol, 1998. 28(9): p. 2902-12.
Taylor, P. A., A. Panoskaltsis-Mortari, J. M. Swedin, P. J. Lucas, R. E. Gress, B. L. Levine, C. H. June, J. S. Serody, and B. R. Blazar. 2004. L-Selectin(hi) but not the L-selectin(lo) CD4+25+ T-regulatory cells are potent inhibitors of GVHD and BM graft rejection. Blood 104:3804.
Thornton, A. M., and E. M. Shevach. 2000. Suppressor effector function of CD4+CD25+ immunoregulatory T cells is antigen nonspecific. J Immunol 164:183.
Tordjman, R., et al., A neuronal receptor, neuropilin-1, is essential for the initiation of the primary immune response. Nat Immunol, 2002. 3(5): p. 477-82.
Vendetti, S., et al., Anergic T cells inhibit the antigen presenting function of dendritic cells. J Immunol, 2000. 165(3): p. 1175-81.
Vieira, P.L., et al., IL-10-secreting regulatory T cells do not express Foxp3 but have comparable regulatory function to naturally occurring CD4+CD25+regulatory T cells. J Immunol, 2004. 172(10): p. 5986-93.
Zhumabekov, T., P. Corbella, M. Tolaini, and D. Kioussis. 1995. Improved version of a human CD2 minigene based vector for T cell-specific expression in transgenic mice. J Immunol Methods 185:133.

### The following items further characterise the present invention:

1. A method of treating an autoimmune disease in an individual comprising:
   administering Neuritin negative T regulatory cells to the individual,
   thereby treating said autoimmune disease in said individual.
2. A method of treating an autoimmune disease in an individual comprising:
   administering CD4+ T cells transduced with a nucleic acid molecule encoding neuritin or a functional fragment thereof, to said individual,
   thereby treating said autoimmune disease in said individual.
3. A method of treating an autoimmune disease in an individual comprising:
   administering an antagonist of Neuritin to said individual, thereby treating said immune disease in said individual.
4. The method of any one of items 1, 2 and 3, further comprising administering to said individual at least one additional therapeutic agent effective in treating said autoimmune disease.
5. A method of treating cancer in an individual comprising:
   administering to said individual lymphocyte infusions containing neuritin positive CD4+ T regulatory cells,
   thereby treating said cancer in said individual.
6. A method of treating cancer in an individual comprising:
   administering tumor specific T cells overexpressing neuritin thereby treating said cancer in said individual.
7. A method of treating cancer in an individual, comprising administering soluble neuritin, or a functional fragment thereof, thereby treating said cancer in said individual.
8. The method of item 7, wherein said soluble neuritin, or functional fragment thereof, is a human Neuritin/Ig chimeric fusion protein
9. The method of any one of items 5, 6 and 7, further comprising administering to said individual at least one additional therapeutic agent effective in treating said cancer.
10. A method of treating an infection in an individual, comprising administering soluble neuritin, or a functional fragment thereof, thereby treating said infection in said individual.
11. The method of item 10, wherein said soluble neuritin, or a functional fragment thereof, is a human Neuritin/Ig chimeric fusion protein.
12. The method of item 10 or item 11, further comprising administering to said individual at least one additional therapeutic agent effective in treating said infection.
13. The method of item 10 or item 11, wherein said infection is a chronic viral infection.
14. A method of enhancing lymphocyte engraftment in an individual wherein said individual has received, is in the process of receiving, or will receive a bone marrow transplant, comprising:
   administering an antagonistic anti-neuritin antibody to said individual, thereby enhancing lymphocyte engraftment.
15. A method of prolonging the persistence of antigen specific T reg cells in an individual, comprising administering a neuritin antagonist to said individual, wherein said antagonist inhibits the immunoregulatory effect of neuritin.
16. The method of item 15, wherein said antagonist is an antibody.
17. An antibody which specifically binds neuritin, wherein said antibody prolongs the lifespan of antigen specific T cells in vivo.
18. The method of item 15, wherein said individual has an autoimmune disease.
19. The method of item 15, wherein said individual has graft vs host disease.
20. A method of preventing the loss of antigen specific T reg cells in an individual, comprising: administering a neuritin antagonist to said individual, wherein said antagonist inhibits the immunoregulatory effect of neuritin.
21. The method of item 20, wherein said antagonist is selected from the group consisting of an antibody, an RNA aptamer, an siRNA molecule, an shRNA molecule, and a fragment of neuritin.
22. An antibody which specifically binds neuritin, wherein said antibody prevents the loss of antigen specific T reg cells in an individual in vivo.
23. The method of item 20, wherein said individual has an autoimmune disease.
24. The method of item 20, wherein said individual has graft vs host disease.
25. A method for reducing the amount antigen specific regulatory T cells in an individual comprising administering a soluble neuritin fusion protein to said individual, wherein said antigen specific regulatory T cells are CD4+ CD25+ T cells, thereby reducing the amount of regulatory T cells.
26. The method of item 25, wherein said neuritin fusion protein is a human Neuritin Ig chimeric molecule.
27. The method of item 25, wherein said neuritin fusion protein is a mouse Neuritin Ig chimeric molecule.
28. A method for reducing the amount of anergized T cells in an individual comprising administering a soluble neuritin fusion protein to said individual.
29. The method of item 28, wherein said neuritin fusion protein is a human Neuritin Ig chimeric molecule.
30. The method of item 28, wherein said neuritin fusion protein is a mouse Neuritin Ig chimeric molecule.
31. The method of item 25 or item 28, wherein said individual has cancer.
32. The method of item 25 or item 28, wherein said antigen specific regulatory T cells are directed to a tumour associated antigen.
33. A method of treating cancer in an individual with cancer comprising administering a soluble neuritin fusion protein to said individual.
34. The method of item 33, wherein said soluble neuritin fusion protein is a human Neuritin Ig chimeric molecule.
35. A method of increasing the efficacy of a vaccine, comprising co-administering soluble neuritin to said individual.
36. The method of item 35, wherein said vaccine is a tumor vaccine.
37. The method of item 35, wherein said vaccine is a vaccine designed to prevent an infectious disease.
38. A method of treating graft versus host disease in an individual comprising administering a neuritin antagonist to said individual, thereby treating said individual.
39. A method of enhancing lymphocyte engraftment after a bone marrow transplantation comprising administering a neuritin antagonist to said individual, thereby enhancing lymphocyte engraftment.
40. A method of modulating an immune response in an individual comprising administering a neuritin antagonist to said individual, thereby treating said individual.
41. A method of modulating an immune response in an individual comprising administering a neuritin antagonist to said individual, thereby modulating an immune response in said individual
42. An isolated antibody, or a fragment thereof, which specifically binds neuritin, wherein said antibody binds human T cell regulatory cells,.
43. The antibody of item 42, wherein said antibody is an antagonistic antibody.
44. The antibody of item 42, wherein said antibody is an agonistic antibody.
45. The antibody of item 42, wherein said antibody is a monoclonal antibody.
46. The antibody of item 42, wherein said monoclonal antibody is selected from the group consisting of 1D6 and 1A9.
47. The antibody of item 42, wherein said antibody is a chimeric antibody.
48. The antibody of item 42, wherein said antibody is a human or humanized antibody.
49. An composition comprising the antibody of item 42 and a carrier.
50. A pharmaceutical composition comprising the antibody of item 42 and a pharmaceutically acceptable carrier.
51. A human Neuritin/Ig chimeric fusion protein.
52. A mouse Neuritin/Ig chimeric fusion protein.
53. A peptide fragment of human neuritin, wherein said fragment inhibits human T cell regulatory cells.
54. A peptide fragment of human neuritin, wherein said fragment activates human T cell regulatory cells.
55. An RNA aptamer directed to human Neuritin.
56. An RNA aptamer directed to mouse Neuritin.
57. The RNA aptamer of item 55, wherein said RNA aptamer specifically inhibits the activity human regulatory T cells.
58. The RNA aptamer of item 55, wherein said RNA aptamer specifically increases the activity of human regulatory T cells.
59. A siRNA molecule which specifically binds to a nucleic acid molecule encoding neuritin.
60. A shRNA molecule which specifically binds to a nucleic acid molecule encoding neuritin.
61. A viral vector comprising a nucleic acid molecule encoding neuritin or a functional fragment thereof, wherein said vector is capable of transfecting an immune cell.
62. The viral vector according to item 61, wherein said vector is a retroviral vector or an adenoviral vector.
63. The vector according to item 61, wherein said immune cell is a T cell.
64. The vector according to item 63, wherein said T cell is a CD4+ T cell.
65. The vector according to item 64, wherein said CD4+ T cell is a T regulatory cell.
66. A polynucleotide encoding the antibody of item 42.
67. An vector comprising the polynucleotide of item 66.
68. The vector of item 67, wherein said vector is an expression vector.
69. A host cell comprising the vector of item 67.
70. A polynucleotide encoding the Neuritin/Ig chimeric fusion protein of item 51.
71. A polynucleotide encoding the Neuritin/Ig chimeric fusion protein of item 52.
72. A vector comprising the polynucleotide of item 70.
73. A vector comprising the polynucleotide of item 71.
74. The vector of item 72, wherein said vector is an expression vector.
75. The vector of item 73, wherein said vector is an expression vector.
76. A host cell comprising the vector of item 74.
77. A host cell comprising the vector of item 75.
78. A polynucleotide encoding the peptide fragment of human neuritin of item 53.
79. A polynucleotide encoding the peptide fragment of mouse neuritin of item 54.
80. An vector comprising the polynucleotide of item 78.
81. An vector comprising the polynucleotide of item 79.
82. The vector of item 80, wherein said vector is an expression vector.
83. The vector of item 81, wherein said vector is an expression vector.
84. A host cell comprising the vector of item 82.
85. A host cell comprising the vector of item 83.
86. A method of treating an inflammatory condition in an individual, comprising administering a neuritin antagonist to said individual, thereby treating said inflammation.
87. The method of item 86, wherein said inflammatory condition is located in the heart.
88. A method of preventing rejection of a transported organ in an individual, comprising administering a neuritin antagonist to said individual, wherein said antagonist inhibits the immunoregulatory effect of neuritin.
89. The method of item 88, wherein said antagonist is an antibody, peptide or small molecule.
90. A conditional neuritin knock out mouse, wherein the CD4+ T cells of said mouse do not express neuritin.
91. A transgenic mouse, wherein the CD4+ CD8- T cells and the CD4- CD8+ T cells of said mouse over-express neuritin.
92. A method of screening for an agent which modulates the immunomodulatory activity of neuritin in vitro comprising:
   co culturing CD4+ T cells, wherein said CD4+T cells comprise a retroviral vector encoding neuritin and wherein said cells carry GFP with bone-marrow derived dendritic cells pulsed with an antigen;
   measuring IL-2 production produced; and
   comparing the amount of IL-2 produced to a control.
93. The method of item 92, wherein the control comprises CD4+ T cells cultured in the absence of said test agent.
94. The method of item 92 and 93, wherein an increase in IL-2 production indicates said agent inhibits the immunomodulatory activity of neuritin.

## Claims

1. An isolated antibody, or a fragment thereof, which specifically binds neuritin, wherein said antibody binds human regulatory T cells.

2. An isolated antibody, or a fragment thereof, according to claim 1, wherein the antibody is
a) an antagonistic antibody,
b) a monoclonal antibody,
c) a monoclonal antibody selected from the group consisting of 1D6 and 1A9,
d) a chimeric antibody, or
e) a human or humanized antibody.

3. A pharmaceutical composition comprising the antibody or fragment thereof, according to claim 1 or 2 and a pharmaceutically acceptable carrier.

4. A neuritin antagonist for use in modulating an immune response in an individual.

5. A neuritin antagonist for use according to claim 4, wherein the neuritin antagonist affects neuritin signaling affecting regulatory T cell homeostasis.

6. A neuritin antagonist for use according to claim 4 or claim 5, wherein the neuritin antagonist reduces regulatory T cells in the individual.

7. A neuritin antagonist for use according to any of claims 4-6, wherein said use comprises modulation of regulatory T cells or immunotherapy of cancer.

8. A neuritin antagonist for the use according to any of claims 4-7, wherein the antagonist is selected from antagonistic antibodies which specifically bind neuritin and inhibit an immunomodulating function of neuritin, neuritin blocking antibodies, RNA aptamers and antagonist peptides, drugs and molecules.

9. A neuritin antagonist for use according to any of claims 4-8, wherein the antagonistic antibody binds to neuritin to block the ability of neuritin to attenuate the potency of antitumor immunity.

10. A neuritin antagonist for use according to any of claims 4-9, wherein said neuritin antagonist is an isolated antibody, or a fragment thereof, which specifically binds neuritin, wherein said antibody binds human regulatory T cells.

11. A neuritin antagonist for use according to any of claims 4-10, wherein said neuritin antagonist is an isolated antibody, or a fragment thereof, and said antibody is:
a) an antagonistic antibody,
b) a monoclonal antibody,
c) a monoclonal antibody selected from the group consisting of 1D6 and 1A9,
d) a monoclonal antibody that binds to the same epitope of the monoclonal antibody 1D6,
e) a chimeric antibody, or
f) a human or humanized antibody.

12. A neuritin antagonist for use according to claim 11(a), wherein said use comprises modulation of regulatory T cells or immunomodulation of cancer.
